# EUROPEAN PATENT SPECIFICATION

(11) **EP 0 955 035 B1**
(45) Date of publication and mention of the grant of the patent: **29.09.2004**
(21) Application number: 99106613.5
(22) Date of filing: 31.03.1999
(51) Int. Cl.: A61K 7/32, A61K 7/16

(54) **Method of preventing aged body odor**
Methode zur Verhütung des Körpergeruchs von alten Leute
Methode pour la prévention des odeurs corporelles de personnes agées

(30) Priority: 31.03.1998 JP 10339998; 31.03.1998 JP 10340098; 31.03.1998 JP 10340198; 31.03.1998 JP 10340298
(43) Date of publication of application: 10.11.1999
(73) Proprietor: SHISEIDO COMPANY LIMITED, Chuo-ku, Tokyo 104-8010 (JP); Takasago International Corporation, Tokyo 144-8721 (JP)
(72) Inventor: Gozu, Yoko, c/o Shiseido Research Center (1), Yokohama-shi, Kanagawa 223-8553 (JP); Haze, Shinichiro, Shiseido Research Center (1), Yokohama-shi, Kanagawa 223-8553 (JP); Nakamura, Shoji, Shiseido Research Center (1), Yokohama-shi, Kanagawa 223-8553 (JP); Saito, Tsutomu, Shiseido Research Center (1), Yokohama-shi, Kanagawa 223-8553 (JP); Sawano, Kiyohito, Takasago Intern. Corp., Tokyo 144-8721 (JP); Yamazaki, Kazuo, c/o Takasago Intl. Corp., Tokyo 144-8721 (JP); Miyasaka, Masataka, Takasago Intl. Corp., Tokyo 144-8721 (JP)
(74) Representative: Henkel, Feiler & Hänzel

(56) References cited:
- EP-A- 0 277 400
- EP-A- 0 340 662
- EP-A- 0 433 911
- DD-A- 293 958
- FR-A- 2 168 422
- FR-A- 2 380 341
- FR-A- 2 666 510
- DATABASE WPI Section Ch, Week 8425 Derwent Publications Ltd., London, GB; Class C84,Page 4387, AN 84009985 XP002113999 & SU 1 001 936 A (URALS CHEM IND RES ), 10 March 1983 (1983-03-10)

## Description

### Field of the Invention

The present invention relates to a method of preventing aged body odors. In particular, the present invention relates to a method of preventing aged body odor containing octenal and/or nonenal.

### Background of the Invention

Body odors can be roughly classified into an "odor of each part of the body", for example, bad breath or halitosis, a foot odor, an armpit odor or a scalp odor, and an "odor given from the overall body".

Hitherto, many studies have been made on the "odor of each part of the body", and the main components of these odors have been analyzed and various excellent means for preventing the odors have been proposed.

For example, sweat, armpit and foot odors are known to be produced from "sweat" by "resident flore of skin".

Sweat is secreted from the sweat glands which can be divided into the eccrine glands which are distributed throughout the body, and the apocrine glands. Sweat secreted from the eccrine glands is called eccrine sweat, which is primarily composed of water but also contains small amounts of sodium chloride, lactic acid, urea and so on. On the other hand, the apocrine glands are present with hair follicles and found only in specific regions such as the fossa axillaris or armpit, the areola mammae and the pubic region. Sweat secreted from the apocrine glands is called apocrine sweat, which is milky and contains proteins, lipids, fatty acids, cholesterol, glucose, ammonia, iron and so on.

Such secreted sweats do not have an offensive odor as they are, but become malodorous due to resident flore of skin which are present on the surface of the skin. In analyses of armpit and foot odors, the lower fatty acids such as pelargonic and capric acids have been detected from the armpit odor, and isovaleric acid has been detected from the foot odor.

At present, as means to prevent the odor of each part of the body, sweat control agents which control sweating which causes the odors, antibacterial agents which inhibit the proliferation of resident flore of skin, odor-eliminating agents which eliminate such substances as causing the odors such as the lower fatty acids, and masking agents which mask the odors using perfumes or fragrances (for example, a fragrant composition for masking, described in Japanese Patent Application Laid-Open No. 6-179610) are used.

As described above, there is a lot of information on the "odor of each part of the body", whereas virtually no studies have been made on the "odor given from the overall body".

Many people feel that human odors change as they age, as evidenced by such expressions as "a baby odor", "an adolescent odor" or "an old people's odor". It is unknown, however, what substances generate such odors and why such odors occur, and no such study has been reported.

On the other hand, people now tend to prefer being "odorless" and hate "smelling", and an odor of people of middle or advanced age, "the aged body odor", is one of the most hated odors at present.

Specifically, the aged body odor is significantly found in people of middle or advanced age, such as those in their forties or over, particularly males, and may be represented as an odor feeling from the nape of the neck or the back of people of middle or advanced age, an odor of a business suit or coat worn by such people, or an odor feeling from a space in which a group of such people are present. In other words, the aged body odor is like an odor from old oil, old soap, old books during the rainy season, or business suits that have been worn for one season and then left without cleaning.

The aged body odor is qualitatively distinctly different from the odor of each part of the body such as armpit, foot or sweat odors, but is as offensive as the odor and has similar adverse effects on people.

The aged body odor actually exists, and it is expected that, in an aging society, there will be an increasing demand for effectively eliminating the aged body odor. The causes of the aged body odor, however, have not been clarified. In addition, it is clear that the means to prevent the odor of each part of the body is insufficient to prevent the generation of the aged body odor or sensoryly prevent it.

Accordingly, an object of the present invention is to clarify the causes of the aged body odor, and to provide a means for preventing the generation of the aged body odor and a means for sensoryly preventing the aged body odor.

The expression "sensoryly preventing the aged body odor" means masking or harmonizing the aged body odor, which leads to the elimination of the offensiveness of the aged body odor.

More specifically, a means for sensoryly preventing the aged body odor includes a means for masking the aged body odor to effectively prevent it and a means for preventing the aged body odor by a harmonizing effect which can harmonize odors as a whole even if the aged body odor is present. The "harmonizing effect" herein means an effect of harmonizing an overall odor including the aged body odor if any, which can eliminate the offensiveness of the aged body odor.

DD-A-293 958 discloses a deodorizing composition containing a lipoxygenase inhibitor.

FR-A-2 666 510 discloses a deodorizing composition containing perfuming components.

EP-A-0 277 400 discloses oral compositions comprising transexamic acid or its salt and an aldehyde flavor.

FR-A-2 168 422 discloses a cosmetic composition comprising a bactericidal compound.

EP-A-0 433 911 discloses a deodorant comprising glycyrrhetic acid.

SU-A-1 001 936 discloses a bath salt containing chlorophyll-carotene paste.

### Summary of the Invention

As a result of enthusiastic studies in order to attain the object, the inventors have found that the aged body odor is not caused by the lower fatty acids which are recognized as typical causal substances of the odor of each part of the body, and, surprisingly, the aged body odor has new characteristics, and closely relates to unsaturated aldehydes such as octenal and nonenal which have not been recognized as a causal substance of body odors.

The inventors had healthy males and females in their twenties to seventies wear washed shirts after bathing, and used the "head space collection method" to allow the TENAX TA resin to adsorb body odors adhering to the shirts. The inventors then desorbed the odors from the resin and analyzed the components of the odors using GC/MS (G1800A GCD System; INNOWAX 60m x 0.25mm column; manufactured by Hewlett Packard).

In addition, the inventors had professional panelists make a sensory evaluation of the body odors of each subject.

The results are shown in Table 1.

**Table 1**

| | Males (n=3) | Females (n=2) | Males (n=3) | Females (n=2) | Males (n=3) | Females (n=2) |
|---|---|---|---|---|---|---|
| | in their twenties to thirties | | in their forties to fifties | | In their sixties to seventies | |
| GC/MS analysis | | | | | | |
| Saturated | | | | | | |
| aldehyde | | | | | | |
| Hexanal | +++ | ++ | + | + | + | + |
| Heptanal | + | + | + | + | + | + |
| Octanal | ++ | ++ | ++ | ++ | ++ | ++ |
| Nonanal | +++ | +++ | +++ | +++ | +++ | +++ |
| Decanal | ++ | + | +++ | +++ | +++ | +++ |
| Unsacuraced | | | | | | |
| aldehyde | | | | | | |
| Octenal | - | - | - | - | + | + |
| Nonenal | - | - | ± | ± | ++ | + |
| Sensoryly | | | | | | |
| evaluation | | | | | | |
| Sweat odor | +++ | ++ | ++ | ++ | + | + |
| Armpit odor | + | + | + | + | + | ± |
| Aged body | - | - | ± | ± | +++ | ++ |
| odor | | | | | | |
| <GS/MS analysis> | | | | | | |
| +++: Detected as a peak 3 times as large as + or larger | | | | | | |
| ++: Detected as a peak 1.5 to 3 times as large as + | | | | | | |
| +: Detected as a peak | | | | | | |
| -: Not detected | | | | | | |
| <Sensory evaluation> | | | | | | |
| +++: The odor is very strongly felt. | | | | | | |
| ++: The odor is strongly felt. | | | | | | |
| +: The odor is felt. | | | | | | |
| ±: The odor is slightly felt. | | | | | | |
| -: The odor is not felt. | | | | | | |

From the results of the sensory evaluation, it was recognized that the aged body odor was distinctly different from the sweat and armpit odors and was specifically detected in subjects of middle or advanced age. In addition, it was recognized that, of the aldehydes detected in GS/MS analysis, unsaturated aldehydes such as octenal and nonenal were such components as increasing with aging.

These results led the inventors to the conclusion that the causal substances of the aged body odor are unsaturated aldehydes such as octenal and nonenal (specifically, 2-octenal and 2-nonenal, in particular, their trans forms).

The inventors then assumed that unsaturated aldehydes are produced due to aging as follows:

It is known that lipoperoxides are produced in the human body by ultraviolet rays or enzymes such as lipoxygenase. For example, it is known that squalene(C₃₀H₅₀) existing in sebum is changed to squalene hydroperoxide (hereinafter also referred to as "SQHPO") by ultraviolet rays (cf. Ohsawa K. et al., J. Toxicol. Sci. 9, 151(1984)). Such produced lipoperoxides are normally decomposed by the biopylactic mechanisms such as a reaction by peroxydase, but the functions of the biophylactic mechanisms tend to decline with aging.

On the other hand, it is also known that the ratio of fatty acids in sebum varies with aging; the ratio of cis-6-hexadecenoic acid which is higher when young, relatively decreases and the ratio of palmitoleic acid (9-hexadecenoic acid) (Journal of Investigative Dermatology 73:112-117, 1979) is increased with aging.

The inventors assumed that the palmitoleic acid is changed to palmitoleic acid hydroperoxide (hereinafter also referred to as "palmitoleic acid HPO") by propagation of oxidization induced by the lipoperoxides (for example, SQHPO) which are not decomposed by the biophylactic mechanisms, and then the palmitoleic acid HPO is β-cleaved to give octenal and nonenal, which cause the aged body odor.

Based on this assumption, the inventors have found that an antioxidant can be used to prevent the generation of the unsaturated aldehydes, which results in preventing not only general body odors but also the aged body odor.

The inventors have also found that a lipoxygenase inhibitor can be used to prevent the generation of the unsaturated aldehydes, which results in preventing the aged body odor.

The inventors further assumed that resident flore of skin are significantly concerned with producing palmitoleic acid in sebum and the palmitoleic acid is changed to palmitoleic acid HPO by propagation of oxidization induced by the lipoperoxides (for example, SQHPO) which are not decomposed by the biophylactic mechanisms, and then the palmitoleic acid HPO is β-cleaved to give octenal and nonenal, which cause the aged body odor.

Based on this assumption, the inventors have found an antibacterial agent can be used to prevent the generation of unsaturated aldehydes such as octenal and nonenal, which results in preventing the aged body odor.

In addition, the inventors studied a means for sensoryly preventing the aged body odor.

As a result, the inventors have found that specific compounds or essential oils conventionally used as perfumes have the desired masking or harmonizing effect on the aged body odor.

Although the inventors have found that unsaturated aldehydes such as octenal and nonenal are concerned with the generation of at least the aged body odor, the means for preventing the aged body odor described above can be used to prevent body odors other than the aged body odor, which the unsaturated aldehydes are concerned with. In this connection, the inventors provide herein not only a means for preventing the aged body odor but also a means for preventing the body odors which result from the unsaturated aldehydes which causes the aged body odor.

Accordingly, first, there is provided a method of preventing aged body odor containing octenal and/or nonenal, comprising the step of applying to the skin and/or hair and/or clothes of an aged person whose body secretes octenal and/or nonenal, and/or introducing into air or space where the aged body odor may be present an effective amount of a composition comprising
(A) at least one selected from the group consisting of an antioxidant, a lipoxygenase inhibitor and an antibacterial agent, and/or
(B) a compound for masking the aged body odor and/or a component for harmonizing the aged body odor.

### Detailed Description of Preferred Embodiments

As described above, the composition to be used according to the present invention contains
(A) at least one selected from the group consisting of an antioxidant, a lipoxygenase inhibitor and an antibacterial agent, and/or
(B) a compound for masking the aged body odor and/or a component for harmonizing the aged body odor.

A. The composition containing at least one selected from the group consisting of an antioxidant, a lipoxygenase inhibitor and an antibacterial agent (which composition hereinafter is also referred to as Composition (A))

### 1. Antioxidant

The type of antioxidant which can be contained in the composition is not particularly limited, provided that it can prevent an oxidization process, through which unsaturated aldehydes are generated, such as oxidization propagation.

Specifically, such an antioxidant includes but is not limited to carotinoids such as α-carotene, β-carotene, γ-carotene, lycopene, cryptoxanthin, zeaxanthin, isozeaxanthin, rhodoxanthin, capsanthin and Crocetin; furans such as 2,5-dimethylfuran, 2-methylfuran, 2,5-diphenylfuran and 1,3-diphenylisobenzofuran; vitamin E such as α-tocopherol, β-tocopherol, γ-tocopherol and δ-tocopherol; amino acids such as histidine, tryptophan, methionine, alanine, and alkylester of alanine; and 1,4-diazabicyclooctane; dibutylhydroxytoluene(BHT), gallates, ascorbic acid, tannins and flavonoid.

Each of these antioxidants can be manufactured using known methods, and of course, a commercially available product may be used in the composition.

In addition, extracts from crude drugs having an effect of inhibiting oxidization can be used as the antioxidant. Specifically, such extracts include but are not limited to Scutellaria root extract, Artemisia capillaris extract, Gambir extract, sage extract, tea extract, rosemary extract, fennel extract, thyme extract, nutmeg extract, pepper extract, turmeric extract, vanilla extract, paprika extract, Coix extract, Bupleurum extract, cucumber extract, Caesalpinia sappam extract, Culen(Psoral Rubescens Pers.) extract and Jungrahab (Baeckea frtescens L.) extract.

The extracts can be prepared by known methods. For example, an extract prepared by extracting a plant with a solvent such as water, an organic solvent or a water-containing organic solvent may be used as it is. Alternatively, a concentrated extract, a purified extract which can be obtained by removing impurities by adsorption or purification using an ion-exchange resin, or an extract concentrated by adsorption and desorption using a column may be also used. An extract prepared by extraction using water/ethyl acetate may be also used, and of course, commercially available plant extracts may be used in the composition.

The amount of the antioxidant in the composition should be determined according to, for example, the type of the antioxidant and the relationship between the antioxidant and the other components, and is not particularly limited. It should, however, be approximately from 0.0001 percent by weight (wt.%) to 10.0 wt.%, preferably from 0.01 wt.% to 1.0 wt.%, base on the total amount of the composition.

In case that the amount of the antioxidant is less than 0.0001 wt.%, the sufficient effect of preventing the aged body odor can not be obtained, which being not preferable. On the other hand, even if it is more than 10.0 wt.%, such increase in the effect of preventing the generation of the body odor as being proportional to the increase in the amount can not be obtained, and, a formulation problem may occur depending on the type of the antioxidant, which being not preferable.

The composition may contain one or more of the antioxidants.

### 2. Lipoxygenase inhibitor

The type of lipoxygenase inhibitor which can be contained in the composition is not particularly limited, provided that it can inhibit the activity of lipoxygenase. The lipoxygenase is an intracellular enzyme which mediates the generation of unsaturated fatty acid peroxides such as linoleic acid HPO, which peroxides induce oxidization propagation to generate the unsaturated aldehydes.

Specifically, such a lipoxygenase inhibitor includes but is not limited to tranexamic acid and β-carotene (β-carotene can be used as a lipoxygenase inhibitor as well as an antioxidant).

The amount of the lipoxygenase inhibitor in the composition should be determined according to, for example, the type of the lipoxygenase inhibitor and the relationship between the lipoxygenase inhibitor and the other components, and is not particularly limited. It should, however, be approximately from 0.0001 wt.% to 10.0 wt.%, preferably from 0.005 wt.% to 5.0 wt.%, based on the total amount of the composition.

In the case that the amount of the lipoxygenase inhibitor is less than 0.0001 wt.%, the sufficient effect of preventing the aged body odor can not be obtained, which being not preferable. On the other hand, even if it is more than 10.0 wt.%, such increase in the effect of preventing the generation of the body odor as being proportional to the increase in the amount can not be obtained, and, a formulation problem may occur depending on the type of the lipoxygenase inhibitor, which being not preferable.

The composition may contain one or more of the lipoxygenase inhibitors.

### 3. Antibacterial agent

The type of antibacterial agent which can be contained in the composition is not particularly limited, provided that it can inhibit resident flore of skin which produce in sebum higher unsaturated fatty acids, in particular, palmitoleic acid, such as Staphylococcus epidermidis, Corynebacterium minutissimum and Malassezia furfur, in particular, Staphylococcus epidermidis.

Specifically, the antibacterial agent includes, for example, trichlorocarbanilide, benzalkonium chloride, benzethonium chloride, halocarban, chlorhexadine hydrochloride, Chamaecyparis pisifera, Chamaecyparis pisifera, propolis, Coptis japonica, Phellodendron amurense, Matricaria chamomilla, Paeonia lactiflora, Hamamelis virginiana and Bupleurum falcatum, and furthermore, dihydrofanesol which is an "antibacterial perfume" having both effects of an antibaterial agent and a perfume (cf. Japanese Patent Application Laid-Open No. 8-245979).

The amount of the antibaterial agent in the composition should be determined according to, for example, the type of the agent and the relationship between the agent and the other components, and is not particularly limited. It should, however, be approximately from 0.0001 wt.% to 10.0 wt.%, preferably from 0.005 wt.% to 5.0 wt.%, based on the total amount of the composition.

In the case that the amount of the antibaterial agent is less than 0.0001 wt.%, the sufficient effect of preventing the aged body odor can not be obtained, which being not preferable. On the other hand, even if it is more than 10.0 wt.%, such increase in the effect of preventing the generation of the body odor as being proportional to the increase in the amount can not be obtained, and, a formulation problem may occur depending on the type of the antibaterial agent, which being not preferable.

The composition may contain one or more of the antibacterial agents.

The composition can be used for preventing the generation of the aged body odor in clothes, which odor is caused by sebum adhering to the clothes which sebum is naturally oxidized to give unsaturated aldehydes, or a means for preventing adhesion of the aged body odor to the clothes, which odor is generated from the body.

The composition can contains at least one antioxidant, at least one lipoxygenase inhibitor and/or at least one antibacterial agent, as a component of preventing the generation of the body odor, and furthermore, if necessary, may contain a component of sensoryly preventing the body odor, which will be described later.

In addition to the component directly related to the prevention of the generation of a body odor resulting from at least unsaturated aldehyde, such as the aged body odor, the composition may contains general components according to the specific embodiment or form, unless the effects of the present invention are lost.

For example, if the composition is used as an external agent for the skin, or toiletries and cosmetic products, the composition may contain as general components, for example,
an ultraviolet absorbent such as p-aminobenzoic acid or p-aminobenzoates; anthranilates such as methyl anthranilate; salicylates such as octyl salicylate, phenyl salicylate or homomenthyl salicylate; cinnamates such as isopropyl p-methoxycinnamate, octyl p-methoxy cinnamate, 2-ethylhexyl 4-methoxycinnamate, glyceryl di-p-methoxycinnamate mono-2-ethylhexanoate or [4-bis (trimethylsiloxy)methylsilyl-3-methylbutyl]-3,4,5-trimethoxycinnamate; benzophenones such as 2,4-dihydroxybenzophenone, 2-hydroxy-4-methoxy-benzophenone, 2-hydroxy-4-methoxybenzophenone-5-sulfonic acid or sodium 2-hydroxy-4-methoxy-benzophenone-5-sulfonate; or urocanic acid, ethyl urocanate, 2-phenyl-5-methylbenzoxazol, 2-(2'-hydroxy-5'-methylphenyl) benztriazole or 4-tert-butyl-4'-methoxy-di-benzoylmethane;
a moisturizer such as polyethylene glycol, propyrene glycol, dipropyrene glycol, 1,3-butylene glycol, hexylene glycol, glycerin, diglycerin, xylitol, maltitol, maltose, D-mannitol, starch syrup, glucose, fructose, lactose, sodium chondroitin sulfate, sodium hyaluronate, sodium adenosine phosphate, sodium lactate, bile salt, pyrrolidonecarboxylic acid, glucosamine or cyclodextrin;
a medical agent, for example, vitamins such as inositol, pyridoxine hydrochloride, benzyl nicotinate, nicotinamide, vitamin D₂(ergocalciferol) or biotin; hormones such as estradiol or ethynylestradiol; antiinflammatory agents such as allantoin, azulene or glycyrrhetinic acid; whitening agents such as arbutin; astringents such as zinc oxide or tannin acid; refrigerants such as L-menthol or camphor; or sulfur or lysozyme chloride; and/or
an extract other than the above plant extracts.

The general components which may be contained in the composition are not limited to the above-mentioned components. Each of the general components, in particular, the medical agents can be contained solely or in combination in the composition according to the embodiment, form or purpose of the composition.

The composition is widely applicable as an external agent for the skin, or toiletries and cosmetic products to various embodiments such as drugs or pharmaceuticals, quasi-drugs (ointment, dentifrice, mouth wash) and cosmetics(basic cosmetics such as facial cleansing foam or cream, lotion, emulsion, cream, gel, essence, pack and mask; makeup cosmetics such as foundation and rouge; oral cosmetics, fragrant cosmetics, hair cosmetics, body cosmetics such as deodorant lotion, powder, spray and stick), but the embodiments of the composition are not limited to these forms.

The composition may be in various forms including aqueous solution, solubilization, emulsion, powder, oil, gel, ointment, aerosol, dispersion, two-layer(water-oil) system and three-layer(water-oil-powder) system.

The composition may contain one or more base components according to the embodiment and form, unless the base component does not lose the effects of the present invention.

The composition may contain one or more of the following base components:
oily components such as liquid or solid fats and oils, wax, esters, hydrocarbons or silicones;
surfactants such as anion, cation, ampholytic, lipophilic nonionic and hydrophilic nonionic surfactants;
water; lower alcohols; sterols; polymers such as vegetable, microbiological, starch, animal, cellulose, alginate, vinyl, acrylic or water-soluble polymers;
sequestering agents; neutralizers and pH regulators.

The above base components are only illustrative, and the base components which may be contained in the composition are not limited to those mentioned above.

The base components to be contained in the composition are appropriately selected according to the embodiment and form of the composition.

The specific formulations of the composition will be described in Example.

In case that the composition is used as a means for preventing generation of the aged body odor in cloths, or, a means for preventing adhesion of the body odor to clothes, the composition may be in such embodiments as a washing finish agent or a coating agent for clothes.

In this case, the composition can contain an. agent which can allow the composition to adhere to the clothes, and other components, according to the purpose of the composition.

The composition in this form is preferably used in such a form as, for example, a liquid or a spray.

B. Composition containing a component having a masking effect on the body odor and/or a component having a harmonizing effect on the body odor (which composition hereinafter is also referred to as Composition (B))

### 1. Component having an effect of masking the body odor

The composition can contain one or more of the essential oils and compounds in Group α, as a component having an effect of masking 'the aged body odor (which component hereinafter is also referred to as the masking component):
Group α: armoise oil, basil oil, galbanum oil, geranium oil, spearmint oil, ylang ylang oil, cardamon oil, elemi oil, lavender oil, peppermint oil, nutmeg oil, chamomile oil, eucalyptus oil, rosemary oil, allylamyl glycolate, 1,1-dimethoxy-2-phenylethane, 2,4-dimethyl-4-phenyltetrahydrofuran, dimethyltetrahydrobenzaldehyde, 2,6-dimethyl-7-octen-2-ol, 1,2-diethoxy-3,7-dimethyl-2,6-octadiene, phenylacetaldehyde, rose oxide and ethyl 2-methylpentanoate.

Of these essential oils and compounds, armoise oil, galbanum oil, geranium oil, spearmint oil, ylang ylang oil, cardamon oil, elemi oil, lavender oil, peppermint oil, chamomile oil, eucalyptus oil, rosemary oil, allylamyl glycolate, dimethyltetrahydrobenzaldehyde, 2,6-dimethyl-7-octen-2-ol, 1,2-diethoxy-3,7-dimethyl-2, 6-octadiene, phenylacetaldehyde, rose oxide and ethyl 2-methylpentanoate have an extremely excellent effect of masking the body and are preferably used.

The amount of the masking component in the composition should be determined according to, for example, the type of the masking component, the masking effect to be applied and the other components, and is not particularly limited.

The composition is provided which has an excellent effect of masking the body odor such as the aged body odor, by using the masking component. The composition is useful in the field of external agents for the skin, or toiletries and cosmetic products.

### 2. Component having an effect of harmonizing the body odor

The composition can contain one or more of the essential oils and compounds in Group β, as a component having an effect of harmonizing the body odor such as the aged body odor (which component hereinafter also refers to as the harmonizing component):

Group β: lavandin oil, lemon oil, lime oil, orange oil, petitgrain oil, bergamot oil, clary sage oil, coriander oil, cypress oil, jasmine absolute, rose oil, rose absolute, 1-(2,6,6-trimethyl-1,3-cyclohexadien-1-yl)-2-buten-1-one, ethyl vanillin, a mixture of 2,6-dimethyl-2-octenol and 3,7-dimethyl-2-octenol, tert-butyl cyclohexylacetate, anisyl acetate, allyl cyclohexyloxyacetate, ethyl linalool, eugenol, coumarin, ethyl acetacetate, 4-phenyl-2,4,6-trimethyl-1,3-dioxane, ethyl acetacetate ethylene glycol ketal, 4-methylene-3,5,6,6-tetramethyl-2-heptanone, ethyl tetrahydrosafranate, geranyl nitrile, cis-3-hexen-1-ol, cis-3-hexenyl acetate, cis-3-hexenyl methyl carbonate, 2,6-dimethyl-5-hepten-1-al, 4-(tricyclo[5.2.1.0^{2.6}]decylidene)-8-butanal, 5-(2,2,3-trimethyl-3-cyclopentenyl)-3-methylpentan-2-ol, p-tert-butyl-α-methylhydrocinnamic aldehyde, ethyl[5.2.1.0^{2.6}]tricyclodecane carboxylate, geraniol, citronellol and citral.

Of these essential oils and compounds, lavandin oil, lemon oil, lime oil, orange oil, petitgrain oil, bergamot oil, cypress oil, jasmine absolute, rose oil, rose absolute, 1-(2, 6, 6-trimethyl-1,3-cyclohexadien-1-yl)-2-buten-1-one, ethyl vanillin, a mixture of 2,6-dimethyl-2-octenol and 3,7-dimethyl-2-octenol, allyl cyclohexyloxyacetate, eugenol, 4-phenyl-2,4,6-trimethyl-1,3-dioxane, 4-methylene-3,5,6,6-tetramethyl-2-heptanon, ethyl tetrahydrosafranate, cis-3-hexen-1-ol, cis-3-hexenyl acetate, 4-(tricyclo[5.2.1.0^{2,6}]decylidene)-8-butanal, p-tert-butyl-α-methylhydrocinnamic aldehyde, ethyl[5.2.1.0^{2,6}]tricyclodecane carboxylate, geraniol and citronellol have an extremely excellent effect of harmonizing the body odor and are preferably used.

The amount of the harmonizing component in the composition should be determined according to, for example, the type of the component, the harmonizing effect to be applied and the other components, and is not particularly limited.

The composition is provided which has an excellent effect of harmonizing the aged body odor, by using the harmonizing component.

The composition containing the masking component and the harmonizing component is extremely excellent and more preferable, because the use of both of the masking component and the harmonizing component in the composition leads to the synergism of the masking effect and the harmonizing effect, therefore the composition has a synergistic effect of sensoryly preventing the body odor.

In this case, the ratio by weight of the masking component to the harmonizing component in the composition is preferably in a range from 1/99 to 80/20 (the masking component/the harmonizing component), more preferably from 3/97 to 60/40, and most preferably from 3/97 to 45/55.

In case that either of the masking component or the harmonizing component is excessive, the excessive component exhibits too strong effect, making it difficult to obtain the desired synergism of the effect of sensoryly preventing the body odor.

The composition preferably contains dihydrofarnesol, in addition to the masking component and/or the harmonizing component, which composition has a more excellent effect of sensoryly preventing the body odor.

As described above, dihydrofarnesol(cf. Japanese Patent Application Laid-Open No. 8-245979) is a perfume having an excellent antibacterial effect. By using this antibacterial perfume in the composition, it is possible to inhibit the decomposition of unsaturated fatty acids by resident flore of skin, so that the generation of the body odor such as the aged body odor can be prevented while the body odor can be sensoryly prevented by the masking and/or harmonizing effect.

Among dihydrofarnesols, (3S)-(6E)-2,3-dihydrofarnesol has an excellent antibacterial effect and is highly fragrant, so that it is preferably used in the composition.

In case of using dihydrofarnesol in the composition, the amount of dihydrofarnesol in the composition should be determined according to, for example, the antibacterial effect to be applied and the other components, and is not particularly limited. The amount of dihyrofarnesol, however, is approximately from 0.01 wt.% to 10.0 wt.%, preferably from 0.1 wt.% to 5.0 wt.%, based on the total amount of the masking component and/or the harmonizing component.

The composition or Composition (B) may furthermore contain one or more components which can be prevent the body odor by preventing the generation of the body odor, such as antioxidants, lipoxygenase inhibitors and antibacterial agents (these components have already described) as another effective component.

In this case, the amount of the component in the composition is not particularly limited, but it is preferably approximately from 0.005 wt.% to 10.0 wt.%, more preferably approximately from 0.01 wt.% to 5.0 wt.%, since the composition further containing approximately from 0.005 wt.% to 10.0 wt.% of the component can more effectively prevent the body odor such as the aged body odor.

The composition or Composition (B) may additionally contain known perfumes and/or conventional additives, provided that the effects of the present invention are not lost.

Known perfumes include hydrocarbons such as α-pinene, limonene and cedrene; aliphatic saturated or unsaturated alcohols such as 1-octen-3-ol and nonanol; saturated or unsaturated chain terpene alcohols such as myrcenol, tetrahydro linalool and nerol; cyclic terpene alcohols such as α-terpineol and bornylmethoxycyclohexanol; sesquiterpene alcohols such as farnesol; aromatic alcohols such as γ-phenylpropyl alcohol and dimethylbenzylcarbinol; aliphatic ketones such as aliphatic aldehyde, terpene aldehyde, aromatic aldehyde and iso-E-super(7-acetyl-1,2,3,4,5,6,7,8-octahydro-1,1,6,7-tetramethylnaphthalene); cyclic terpene ketones; cyclic ketones; esters of aliphatic acids such as acetate, propionate, butyrate, benzoate, phenylacetate, salicylate and anthranilate. The amount of the perfume in the composition should be decided according to, for example, the type of the perfume and the desired purpose, and is not particularly limited.

Conventional additives include known ultraviolet absorbents, refrigerants such as menthol and methyl salicylate, and skin stimulants such as Capsicum tincture and nonanyl vanillylamide. The amount of the conventional additive in the composition should be decided according to, for example, the type of the perfume and the desired purpose, and is not particularly limited.

The composition is stable, and, as described above, can contain various known perfumes in a wide range of amount, so that it can be used as a perfume for preparation of various products.

The composition can be used as external agents for the skin, but preferably used as toiletries and cosmetic products.

In this case, the amount of the conventional additives in the composition is not particularly limited, but should be approximately from 0.005 wt.% to 5.0 wt.%, based on the total amount of the composition.

The composition or Composition B may contain one or more base components according to the embodiment and form unless the base component does not lose the effects of the present invention (Exemplary base components have been already mentioned).

The composition can be also incorporated in a formulation for preparing toiletries and cosmetic products. The toiletries and cosmetic products containing the composition are also an embodiment of a composition of the present invention.

The toiletries and cosmetic products of the present invention include, for example, perfumes, eau de Cologne, eau de Toilette, deodorant sprays, soaps, shampoos, rinses, lotions, body shampoos, and aromatics. The toiletries and cosmetic products can contain the composition in a wide range of amount, and preferably in an amount approximately from 0.1 wt.% to 20.0 wt.%, based on the total amount thereof, but the amount should not be limited.

Furthremore, in order to provide an agent for sensoryly preventing the body odor such as the aged body odor adhering to clothes, the composition can be in a form of a washing finish agent or a coating agent for clothes. The composition can be also incorporated in a washing finish agent or a coating agent for clothes (The washing finish agent and the coating agent for clothes are also an embodiment of the composition of the present invention).

"For preventing the aged body odor" herein is novel use and should be clearly distinguished from and independent of "for preventing general body odors" for the following reasons:
i) "the aged body odor" results from substances which are obviously different from the causal substances of the general body odors such as sweat and armpit odors (described above);
ii) "the aged body odor^{"} is generated independently of the general body odors and generally in people of middle or advanced age;
iii) it is difficult to prevent "the aged body odor" using the conventional means for preventing the general body odors (described below).

Therefore, "for preventing the aged body odor" should be clearly distinguished from "for preventing the eneral body odors" in terms of techniques and concepts.

### Examples

This invention will be specifically described below using examples, but these examples are not intended to limit the scope of the present invention.

It should be noted that the amount is represented in terms of the weight percentage of the total amount of the composition into which the component is incorporated, unless otherwise specified.

### A. Examples of Composition (A)

### Test Example A-I-1

### Aged body odor generation test using squalene hydroperoxide

As described above, it is known that squalene existing in sebum is subjected to peroxidation by ultraviolet rays to generate squalene hydroperoxide (SQHPO) (cf. Ohsawa K., et al., J. Toxicol. Sci. 9. 151(1984)), but there has been no report on the relationship between SQHPO and the generation of the body odor, particularly the aged body odor.

Therefore, in order to prove the above-described hypothesis, the relationship between the reaction of fatty acids in sebum, particularly unsaturated fatty acids, with SQHPO and the generation of the aged body odor was investigated.

Specifically, the causes of the generation of the aged body odor were examined using hexadecenoic acid (palmitoleic acid, C16: 1) and an octadecenoic acid (C18: 1), which are the main unsaturated fatty acids found in sebum, as well as SQHPO.

### Test method

Squalene hydroperoxide (SQHPO) was prepared by irradiating squalene to which Methylene Blue had been added as a sensitizer, with light from a mercury lamp, and separating generated SQHPO by liquid chromatography. The structure of the SQHPO was confirmed using NMR.

In vials were weighed 1 mg of cis-9-hexadecenoic acid (manufactured by Tokyo Kasei Co., Ltd.; hereinafter referred to as "hexadecenoic acid" unless otherwise specified), 1 mg of cis-9-octadecenoic acid (manufactured by Tokyo Kasei Co., Ltd.; hereinafter referred to as "octadecenoic acid" unless otherwise specified) , and 0.01 mg of SQHOP, and then concentrated to dryness by a centrifugal concentrator, and left at 37°C.

After maintaining the temperature for seven days, the samples were sensoryly evaluated for odors to determine whether the aged body odor had been generated, and GC/MS (G1800A GCD System, Innowax 60 x 0.25 mm column; manufactured by Hewlett Packard) was used to analyze the samples for aldehydes which were assumed to cause the aged body odor.

The results of the test are shown in Table A1.

**Table A1**

| Sample | Aged body odor | Detected aldehyde |
|---|---|---|
| Only Octadecenoic acid | Absent | Absent |
| Octadecenoic acid + SQHPO | Absent | Nonanal, Pentenal |
| Only Hexadecenoic acid | Present (trace) | Octenal (trace), Nonenal (trace) |
| Hexadecenoic acid + SQHPO | Present | Hexanol, Heptanal, Octenal, Nonenal |

As seen from Table A1, when SQHPO was added to hexadecenoic acid, an odor very similar to the aged body odor was generated. However, the aged body odor was not perceived when SQHPO was added to octadecenoic acid.

These results show that oxidization propagation occurs from SQHPO to hexadecenoic acid, so that hexadecenoic acid is decomposed to give unsaturated aldehydes such as octenaol and nonenal, which are odor components characteristic of the aged body odor. In case of only hexadecenoic acid, a trace of generation of the aged body odor was perceived, which was caused by natural oxidization.

Obviously, these results experimentally prove the above hypothesis on the generation of the aged body odor.

### Test Example A-I-2

### Aged body odor preventing test using antioxidant

Test example A-I-1 clearly showed that SQHPO generated from squalene in sebum due to UV causes oxidization propagation, thereby fatty acids in sebum are decomposed to generate unsaturated aldehydes, which aldehydes cause the aged body odor.

A test was conducted in which an antioxidant was used to prevent the generation of the hydroperoxide as it is or interrupt the oxidization propagation from the hydroperoxide, to determine whether the generation of the aged body odor was prevented.

BHT(dibutylhydroxytoluene) and Culen extract [Culen is a plant which grows in dry pampas and pastures in South America, particularly, the Andes; in this test was used the extract prepared by extracting the entire plant by a conventional method (the specific extraction method will be described below)] were used as antioxidants to conduct the following tests.

### Test Method

In vials were weighed 1 mg of hexadecenoic acid (manufactured by Tokyo Kasei Co., Ltd.), 0.1 mg of SQHPO manufactured as in Test Example A-I-1, and 1 mg of each antioxidant, and then concentrated to dryness using a centrifugal concentrator, and left at 37°C.

After maintaining the temperature for seven days, the samples were sensoryly evaluated for odors to determine whether the aged body odor had been generated, and were analyzed using the GC/MS (G1800A GCD System, Innowax 60 x 0.25 mm column; manufactured by Hewlett Packard) for aldehyde.

The results of the test are shown in Table A2.

**Table A2**

| | Aged body odor | Detected aldehyde |
|---|---|---|
| Only Hexadecenoic acid | Present (trace) | Octenal (trace), Nonenal(trace) |
| Hexadecenoic acid + BHT | Absent | Absent |
| Hexadecenoic acid + Culen extract | Absent | Absent |
| Hexadecenoic acid + SQHPO | Present | Hexanal, Heptanal, Octenal, Nonenal |
| Hexadecenoic acid + SQHPO + BHT | Absent | Absent |
| Hexadecenoic acid + SQHPQ + Culen extract | Absent | Absent |

The results in Table A2 shows that the addition of the antioxidant prevents the generation of the unsaturated aldehydes, thereby the generation of the aged body odor can be prevented.

That is, it was clarified that the addition of the antioxidant prevents the generation of unsaturated aldehydes such as octenal or nonenal, which are normally generated by the oxidization propagation, therefore, the composition containing an antioxidant has an effect of preventing the body odor such as the aged body odor.

### Test Example A-II-1

### Aged body odor generation test using lipoxygenase

Lipoxygenase (also referred to as lipoxydase) is an enzyme which introduces an oxygen molecule into an unsaturated fatty acid having a 1-cis- or 4-cis-pentadiene structure, to generate the hydroperoxide. It has recently been reported that lipoxygenase exists on the human skin and is concerned with the generation of lipoperoxide [cf. Takahashi Y., et al., J. Biol. Chem., 268, 16443(1993), etc.], but there has been no report on the relationship between lipoxygenase and the generation of the body odor such as the aged body odor.

Therefore, in order to prove the above-described hypothesis, an aged body odor generation test was conducted in vitro, noticing the reaction between fatty acids in the skin and lipoxygenase.

Specifically, the causes of the generation of the aged body odor were investigated using linoleic acid (Sigma) and cis-9-hexadecenoic acid (palmitoleic acid; C16: 1), which are primary unsaturated fatty acids in sebum, as substrates.

### Test Method

To a 0.2M borate buffer (pH 9.0) were added either or both of linoleic acid (Sigma) and cis-9-hexadecenoic acid (Tokyo Kasei Co., Ltd.) as a substrate up to 0.2 mg/mL of the concentration of each acid in the buffer, and then added soybean lipoxygenase (SERVA;126,000 U/mg) up to 500 U/mL. The resultant mixture was reacted at 23°C overnight.

After completion of the lipoxygenase reaction, lipid was extracted from each sample using diethyl ether, dried up, and then left at 37°C. After maintaining the temperature for four days, the samples were sensoryly evaluated for odors to determine whether the aged body odor had been generated, and the GC/MS (G1800A GCD System, Innowax 60 x 0.25 mm column; manufactured by Hewlett Packard) was used to analyze the samples for aldehydes which were assumed to cause the aged body odor.

The results of the test are shown in Table A3.

**Table A3**

| Substrate | Aged body odor | Detected aldehyde |
|---|---|---|
| Only Linoleic acid | Absent | Hexanal |
| Only Hexadecenoic acid | Absent | Absent |
| Linoleic acid + Hexadecenoic acid | Present | Hexanal, Heptanal, Octenal, Nonenal |

As seen from Table A3, when both of linoleic acid and hexadecenoic acid were used as substrates, an odor very similar to the aged body odor was generated. On the other hand, when only either of linoleic acid or hexadecenoic acid was used as a substrate, no aged body odor was perceived.

It is assumed that this phenomenon occurs as follows: Lipoxygenase generates linoleic acid hydroperoxide from linoleic acid which has a 1-cis- and 4-cis-pentadiene structure, as a substrate, and the oxidation propagation occurs from linoleic acid hydroperoxide to hexadecenoic acid, thereby hexadecenoic acid is decomposed to generate unsaturated aldehydes such as octenal and nonenal, which are odor components characteristic of the aged body odor.

Obviously, these results have experimentally proved the hypothesis on the generation of the aged body odor.

### Test Example A-II-2

### Aged body odor preventing test using lipoxygenase inhibitor

### 1. Evaluation test on lipoxygenase inhibiting effect of lipoxygenase inhibitor

Test Example A-II-1 has suggested the possibility that oxidization propagation actually occurs in sebum from the hydroperoxide which is generated by lipoxygenase, thereby fatty acids in sebum are decomposed to generate unsaturated aldehydes which cause the aged body odor.

Therefore, an evaluation test was conducted as to whether or not a lipoxygenase inhibitor effectively inhibits the reaction caused by lipoxygenase, as a means of preventing the generation of the aged body odor.

### Test Method

To 0.2M borate buffer (pH 9.0) was added, as a substrate, linoleic acid (Sigma) up to 0.2 mg/mL, and then added soybean lipoxygenase (SERVA;126,000 U/mg) up to 500 U/mL. The resultant mixture was placed in a dissolved-oxygen measuring chamber(Able Corp.), and the temperature was maintained at 23°C. A dissolved-oxygen electrode (Able Corp.) was used to monitor the consumption of dissolved oxygen caused by lipoxygenase reaction. Under such monitoring, one of a series of test agents (in which β-carotene or tranexamic acid has various concentrations) was added to the reaction solution while the amount of dissolved oxygen was decreasing at a constant speed, to investigate the prevention of the consumption of the dissolved oxygen by the inhibition of the lipoxygenase reaction.

### Test Results

As a result of the test, of various agents believed to have a lipoxygenase inhibiting effect, β-carotene and tranexamic acid exhibited a lipoxygenase inhibiting effect in concentration ranges between 0.1 and 10 µg/mL and between 0.1 and 10 mg/mL, respectively.

### 2. Aged body odor generation preventing test using lipoxygenase inhibitor

Based on the results of the above test, β-carotene and tranexamic acid were used as lipoxygenase inhibitors to examine the effect of preventing the generation of the aged body odor caused by lipoxygenase.

### Test Method

To a 0.2 M borate buffer (pH 9.0) was added, as a substrate, linoleic acid (Sigma) or cis-9-hexadecenoic acid (Tokyo Kasei Co., Ltd.) up to 0.2 mg/mL, and soybean lipoxygenase (SERVA; 126,000 U/mg) up to 500 U/mL. Then, to the resultant mixture was β-carotene (final concentration: 1 µg/mL) or tranexamic acid (final concentration: 1 mg/mL), and the mixture was reacted at 23°C overnight.

After completion of the lipoxygenase reaction, lipid was extracted from the reaction mixture using diethyl ether, dried up, and then left at 37°C. After the temperature was maintained for 4 days, the samples were sensoryly evaluated for odors, to determine whether the aged body odor had been generated, and in addition, each dried sample was dissolved in diethyl ether and analyzed using GC/MS (G1800A GCD System, Innowax 60 x 0.25 mm column; manufactured by Hewlett Packard) for aldehyde.

The results of the test are shown in Table A4.

**Table A4**

| Lipoxygenase inhibitor | Aged body odor | Detected aldehyde |
|---|---|---|
| None | Present | Hexanal, Heptanal, Octenal, Nonenal |
| β-Carotene | Absent | Absent |
| Tranexamic acid | Absent | Absent |

The results in Table A4 shows that the addition of the lipoxygenase inhibitor prevents the generation of unsaturated aldehydes, to enable the generation of the aged body odor to be prevented.

That is, it was clarified that the addition of the lipoxygenase inhibitor prevents the generation of unsaturated aldehydes such as octenal and nonenal, which are normally generated by oxidization propagation, etc., therefore, the composition containing a lipoxygenase inhibitor can prevent the generation of the body odor such as the aged body odor.

### Test Example A-III-1

### Aged body odor generation test using microorganism

Resident flore of skin are related to the generation of odors unique to individual regions, such as sweat odor, foot odor, armpit odor, and scalp or dandruff odor. That is, for example, Staphylococcus epidermidis, Corynebacterium minutissimum and Malassezia furfur are believed to be related to the generation of foot odor, armpit odor and dandruff odor, respectively. Such bacteria are known to decompose sebum or sweat using enzymes such as lipase or esterase, thereby lower fatty acids which are odor components characteristic of foot, armpit or dandruff odor, such as isovaleric acid or 3-methyl-2-hexene acid, are generated. However, there has been no report on the relationship between resident flore of skin and the generation of the aged body odor.

Therefore, in order to prove the above-described hypothesis, an aged body odor generation test was conducted in vitro, noticing the relationship between the generation of the aged body odor and the resident flore of skin.

### Test method

Each (one platinum loop) of Staphylococcus epidermidis(SE), Corynebacterium minutissimum(CM) and Malassezia furfur(MF) was inoculated from a slant culture medium to 5mL of a sensitive bouillon culture medium, and then cultured with shaking at 37°C for 24 hours. Following the incubation, the bacteria were collected by centrifugation, washed with a 0.1 M phospate buffer(pH 7.4) and resuspended in 10 mL of a fresh 0.1 M phospate buffer(pH 7.4) to obtain a test bacteria solution.

In a 200 mL Erlenmeyer flask were placed 30 mL of 0.1 M phospate buffer(pH 7.4), 1 mL of cis-9-hexadecenoic acid(Tokyo Kasei Co., Ltd.) and 10 mL of each test bacteria solution, and were cultured with shaking at 37°C for 5 days. After completion of the incubation, extraction was carried out using diethyl ether under an acid condition with sulfuric acid, and the extract was dehydrated using sodium sulfate anhydride, and then concentrated. The resultant concentrates were subjected to the sensory evaluation and the GC/MS analysis

The results of the test are shown in Table A5.

**Table A5**

| | Aged body odor | Detected Aldehyde |
|---|---|---|
| SE/Hexadecenoic acid | Present | Heptanal, Heptenal, Octenal, Nonenal |
| CM/Hexadecenoic acid | Present(weak) | Heptanal, Octenal, Nonenal |
| MF/Hexadecenoic acid | Present(trace) | Octenal(trace), Nonenal(trace) |

The results of Table A5 shows that the microorganisms existing on the skin decompose hexadecenoic acid to generate the aged body odor. Furthermore, it was confirmed that aldehydes such as octenal and nonenal, which are components characteristic of the aged body odor, had been generated.

Obviously, these results experimentally prove the hypothesis on the generation of the aged body odor.

### Test Example A-III-2

### Aged body odor preventing test using antibacterial agent

Since Test Example A-III-1 suggests that microorganisms existing on the skin decompose sebum to generate the aldehydes which are components characteristic of the aged body odor, the effect of preventing the aged body odor by antibacterial agents was examined.

### Test Method

Trichlorocarbanilide, benzalkonium chloride and dihydrofarnesol were used as antibacterial agents, to examine the effect of preventing the aged body odor.

That is, one platinum loop of Staphylococcus epidermidis(SE) was inoculated from a slant culture medium into a sensitive bouillon culture medium and cultured with shaking at 37°C for 24 hours.

Following the incubation, bacteria were collected by centrifugation, washed with a 0.1 M phospate buffer (pH 7.4), and then resuspended in 10 mL of a fresh 0.1 M phospate buffer(pH 7.4), to obtain a test bacteria solution. In a 200 mL Erlenmeyer flask were placed 30 mL of a 0.1 M phospate buffer(pH 7.4), 1 mL of cis-9-hexadecenoic acid (Tokyo Kasei Co., Ltd.) and 10 mL of a test bacteria solution, and then to the mixture was added trichlorocarbanilide, benzalkonium chloride or dihydrofarnesol up to 0.05 wt.%. The resultant mixture was then cultured with shaking at 37°C for 5 days.

In this test, there was used as dihydrofarnesol the. dihydrofarnesol(3,7,11-trimethyl-6,10-dodecadien-1-ol) synthesized from all-trans-nerolidol.

After completion of the incubation, extraction was carried out using diethyl ether under an acid condition with sulfuric acid. Then, the extract was dehydrated using sodium sulfate anhydride, and then concentrated. The resultant concentrates were subjected to the sensory evaluation and the GC/MS analysis.

The results of the test are shown in Table A6.

**Table A6**

| Antibacterial agent | Aged body odor | Detected Aldehyde |
|---|---|---|
| None | Present | Heptanal, Heptenal, Octenal, Nonenal |
| +Trichlorocarbanilide | Absent | Absent |
| +Benzalkonium chloride | Absent | Absent |
| +Dihydrofarnesol | Absent | Absent |

The results in Table A6 shows that the addition of the antibacterial agent prevents the decomposition of fatty acids caused by microorganisms, to prevent the generation of the aged body odor.

Next, we will examine the composition of the present invention in a case where the composition is in a form of an external agent for the skin, which is a representative embodiment of the present composition.

That is, the formulations of the composition of the present invention as an external agent for the skin will be described together with those of the comparative examples. The sensory evaluation of these external agent was conducted as described below.

### Sensory evaluation method of external agent for skin

Subjects of middle to advanced age (in their fifties to seventies) who were selected by professional panelists as strongly having a body odor characteristic of people of middle or advanced age (the aged body odor), were divided into two groups, one group using an agent for preventing the body odor according to the present invention, and the other group using an agent shown in comparative examples.

Each group used the agent each day after or on bathing (sprayed the agent over the entire body).

After the agent was used for three days, the professional panelists conducted the sensory evaluation and evaluated the strength of the body odor of each subjects. The results of the sensory evaluation are represented using the following marks:

### Sensory Evaluation

ⓞ: Scale of strength is none.
○: Scale of strength is slight.
Δ: Scale of strength is medium.
×: Scale of strength is strong.

### Example A1 Lotion

| Components | Amount (wt.%) |
|---|---|
| (1) Glycerine | 4.0 |
| (2) 1,3-Butylene glycol | 4.0 |
| (3) Ethanol | 7.0 |
| (4) Polyoxyethylene(18) oleyl ether | 0.5 |
| (5) Citric acid | 0.05 |
| (6) Sodium citrate | 0.1 |
| (7) Trisodium ethylenediaminetetraacetate | 0.02 |
| (8) Culen extract | 0.5 |
| (9) Purified water | Residue |

### <Preparation method>

Culen extract was prepared by adding 500 mL of water to 500 g of chopped Culen, extracting at 50°C for 60 minutes, cooling, naturally filtering using a filter paper, and to the filtrate, adding purified water up to 500 mL of the total volume.

An aqueous phase was prepared by dissolving citric acid, sodium citrate, glycerine, 1,3-butylene glycol, Culen extract and trisodium ethylenediaminetetraacetate in purified water.

Separately, an ethanol phase was prepared by dissolving polyoxyethylene(18) oleyl ether in ethanol. The ethanol phase was then added to the aqueous phase and the mixture was solubilized, and filtered to obtain the desired lotion.

A similar preparation was carried out without component(8), to obtain the lotion in Comparative Example A1.

### <Test results>

The lotions in Example A1 and Comparative Example A1 were subjected to the sensory evaluation, and it was found that the group using the lotion in Example A1 showed a significant decrease in the body odor compared to the group using the lotion in Comparative Example A1.

The results are shown in Table A7.

**Table A7**

| Subject No. | Used Composition | Body odor after three days |
|---|---|---|
| 1 | Example A1 | ○ |
| 2 | Example A1 | ○ |
| 3 | Example A1 | ⓞ |
| 4 | Example A1 | ⓞ |
| 5 | Example A1 | ○ |
| 6 | Comparative Example A1 | × |
| 7 | Comparative Example A1 | Δ |
| 8 | Comparative Example A1 | Δ |
| 9 | Comparative Example A1 | × |
| 10 | Comparative Example A1 | Δ |

### Example A2 Emulsion

| Components | Amount (wt.%) |
|---|---|
| (1) Stearic acid | 1.5 |
| (2) Cetyl alcohol | 0.5 |
| (3) Beeswax | 2.0 |
| (4) Polyoxyethylene(10) monooleate | 1.0 |
| (5) Octyl methoxycinnamate | 2.0 |
| (6) Scutellaria root extract | 0.2 |
| (7) Sodium hyaluronate | 0.01 |
| (8) Triethanolamine | 0.075 |
| (9) Glycerine | 7.0 |
| (10) Inositol | 0.5 |
| (11) Sodium ethylenediaminehydroxytriacetate | 0.01 |
| (12) Purified water | Residue |

### <Preparation method>

Scutellaria root extract was prepared by adding 500 mL of purified water to 500 g of chopped Scutellaria baicalensis Georgi root with no periderm, extracting at 50°C for 60 minutes, cooling, naturally filtering with a filter paper, and to the filtrated, adding purified water up to 500 mL of the total volume.

An aqueous phase was prepared by dissolving Scutellaria root extract, sodium hyaluronate, glycerine, inositol and sodium ethylenediaminehydroxytriacetate in purified water, and maintaining at 70°C.

Separately, a oil phase was prepared by mixing the other components together, heating and dissolving, and then maintaining at 70° C.

The oil phase was then added to the aqueous phase, the mixture was subjected to preliminary emulsification, and then emulsified using a homogenizing mixer and then quenched while being stirred to obtain the desired emulsion.

A similar preparation was carried out without component(6) to obtain the emulsion in Comparative Example A2.

The emulsions in Example A2 and Comparative Example A2 were fully applied to the upper part of the body, so that the emulsion in Example A2 significantly reduced the body odor of the subjects compared to the emulsion in Comparative Example A2.

### Example A3 Lotion spray

| Components | Amount (wt.%) |
|---|---|
| (Solution formulation) | |
| (1) Zinc p-phenolsulfonate | 2.0 |
| (2) Ethanol | 92.8 |
| (3) 1,3-Butylene glycol | 3.0 |
| (4) Isopropyl myristate | 2.0 |
| (5) Bupleurum extract | 0.2 |

| (Filling formulation) | |
|---|---|
| (6) Solution | 50.0 |
| (7) LPG | 50.0 |

### <Preparation method>

Bupleurum extract was prepared by adding 500 mL of purified water to 500 g of chopped root of Bupleurum falcatum L., extracting at 50°C for 60 minutes, cooling, naturally filtering with a filter paper, and to the filtrate, adding purified water up to 500 mL of the total volume.

The solution was prepared by dissolving in ethanol the other components of the solution, and filtering. The solution obtained was then placed in an aerosol container. A valve was then installed in the container and LPG was charged therein to obtain the desired lotion spray.

A similar preparation was carried out without component(5) to obtain the lotion spray in Comparative Example A3.

### <Test results>

The lotion sprays in Example A3 and Comparative Example A3 were subjected to the sensory evaluation, so that it was found that the group using the lotion spray in Example A3 showed a significant decrease in the body odor compared to the group using the lotion spray in Comparative Example A3.

### Example A4 Lotion

| Components | Amount(wt.%) |
|---|---|
| (1) Glycerine | 4.0 |
| (2) 1,3-Butylene glycol | 4.0 |
| (3) Ethanol | 7.0 |
| (4) Polyoxyethylene(18)oleyl ether | 0.5 |
| (5) Citric acid | 0.05 |
| (6) Sodium citrate | 0.1 |
| (7) Trisodium ethylenediaminetetraacetate | 0.02 |
| (8) Vitamin E | 0.5 |
| (9) Dihydrofarnesol | 0.1 |
| (10) Purified water | Residue |

### <Preparation method>

An aqueous phase was prepared by dissolving citric acid, sodium citrate, glycerine, 1,3-butylene glycol and trisodium ethylenediaminetetraacetate in purified water.

Separately, an ethanol phase was prepared by dissolving polyoxyethylene (18) oleyl ether, vitamin E and dihydrofarnesol in ethanol.

The ethanol phase was then added to the aqueous phase and the mixture was solubilized, and filtered to obtain the desired lotion.

A similar preparation was carried out without components (8) and (9) to obtain the lotion in Comparative Example A4 (the same lotion in Comparative Example A1).

### <Test results>

The lotions in Example A4 and Comparative Example A4 were subjected to the sensory evaluation, so that it was found that the group using the lotion in Example A4 showed a significant decrease in the body odor compared to the group using the lotion in Comparative Example A4.

The results are shown in Table A8.

**Table A8**

| Subject No. | Used Composition | Body odor after three days |
|---|---|---|
| 1 | Example A4 | ○ |
| 2 | Example A4 | ○ |
| 3 | Example A4 | ⓞ |
| 4 | Example A4 | ⓞ |
| 5 | Example A4 | ⓞ |
| 6 | Comparative Example A4 | × |
| 7 | Comparative Example A4 | Δ |
| 8 | Comparative Example A4 | Δ |
| 9 | Comparative Example A4 | × |
| 10 | Comparative Example A4 | Δ |

### Example A5 Lotion

| Components | Amount (wt.%) |
|---|---|
| (1) Glycerine | 4.0 |
| (2) 1,3-Butylene glycol | 4.0 |
| (3) Ethanol | 7.0 |
| (4) Polyoxyethylene(18) oleyl ether | 0.5 |
| (5) Citric acid | 0.05 |
| (6) Sodium citrate | 0.1 |
| (7) Trisodium ethylenediaminetetraacetate | 0.02 |
| (8) Tranexamic acid | 0.5 |
| (9) Purified water | Residue |

### <Preparation method>

An aqueous phase was prepared by dissolving citric acid, sodium citrate, glycerine, 1,3-butyleneglycol, tranexamic acid and trisodium ethylenediaminetetraacetate in purified water.

Separately, an ethanol phase was prepared by dissolving polyoxyethylene (18) oleyl ether in ethanol.

The ethanol phase was then added to the aqueous phase and the mixture was solubilized and filtered to obtain the desired lotion.

A similar preparation was carried out without component (8) to obtain the lotion in Comparative Example A5 (the same lotion in Comparative Example A1).

### <Test results>

The lotions in Example A5 and Comparative Example A5 were subjected to the sensory evaluation, so that it was found that the group using the lotion in Example A5 showed a significant decrease in the body odor compared to the group using the lotion in Comparative Example A5.

The results are shown in Table A9.

**Table A9**

| Subject No. | Used Composition | Body odor after three days |
|---|---|---|
| 1 | Example A5 | ○ |
| 2 | Example A5 | ○ |
| 3 | Example A5 | ⓞ |
| 4 | Example A5 | ⓞ |
| 5 | Example A5 | ○ |
| 6 | Comparative Example A5 | × |
| 7 | Comparative Example A5 | Δ |
| 8 | Comparative Example A5 | Δ |
| 9 | Comparative Example A5 | × |
| 10 | Comparative Example A5 | Δ |

### Example A6 Emulsion

| Components | Amount(wt.%) |
|---|---|
| (1) Stearic acid | 1.5 |
| (2) Cetyl alcohol | 0.5 |
| (3) Beeswax | 2.0 |
| (4) Polyoxyethylene(10) monooleate | 1.0 |
| (5) Octyl methoxycinnamate | 2.0 |
| (6) β-Carotene | 0.2 |
| (7) Sodium hyaluronate | 0.01 |
| (8) Triethanolamine | 0.75 |
| (9) Glycerine | 7.0 |
| (10) Inositol | 0.5 |
| (11) Sodium ethylenediaminehydroxytriacetate | 0.01 |
| (12) Purified water | Residue |

### <Preparation method>

An aqueous phase was prepared by dissolving sodium hyaluronate, glycerine, inositol and sodium ethylenediaminehydroxytriacetate in purified water, and maintaining at 70°C.

Separately, an oil phase was prepared by mixing the other components together, heating and dissolving, and then maintaining at 70°C.

The oil phase was then added to the aqueous phase and the mixture is subjected to preliminary emulsification, and then emulsified using a homogenizing mixer and then quenched while being stirred to obtain the desired emulsion.

A similar preparation was carried out without component(8) to obtain the emulsion in Comparative Example A6.

The emulsions in Example A6 and Comparative Example A6 were fully applied to the upper part of the body, so that the emulsion in Example A6 significantly reduced the body odor of the subjects compared to the emulsion in Comparative Example A6.

### Example A7 Lotion spray

| Components | Amount(wt.%) |
|---|---|
| (Solution formulation) | |
| (1) Zinc p-phenolsulfonate | 2.0 |
| (2) Ethanol | 92.6 |
| (3) 1,3-Butylene glycol | 3.0 |
| (4) Isopropyl myristate | 2.0 |
| (5) Tranexamic acid | 0.2 |
| (6) β-Carotene | 0.2 |

| (Filling formulation) | |
|---|---|
| (7) Solution | 50.0 |
| (8) LPG | 50.0 |

### <Preparation method>

First, the solution was prepared by dissolving in ethanol the other components of the solution and filtering. The solution obtained was then placed in an aerosol container. A valve was then installed in the container and LPG was charged therein to prepare the desired lotion spray.

A similar preparation was carried out without components(5) and (6) to obtain the lotion spray in Comparative Example A7.

### <Test results>

The lotion sprays in Example A7 and Comparative Example A7 were subjected to the sensory evaluation, so that it was found that the group using the lotion spray in Example A7 showed a significant decrease in the body odor compared to the group using the lotion spray in Comparative Example A7.

### Example A8 Lotion

| Components | Amount(wt.%) |
|---|---|
| (1) Glycerine | 4.0 |
| (2) 1,3-Butylene glycol | 4.0 |
| (3) Ethanol | 7.0 |
| (4) Polyoxyethylene(18) oleyl ether | 0.5 |
| (5) Citric acid | 0.05 |
| (6) Sodium citrate | 0.1 |
| (7) Trisodium ethylenediaminetetraacetate | 0.02 |
| (8) β-Carotene | 0.5 |
| (9) Dihydrofarnesol | 0.1 |
| (10) Purified water | Residue |

### <Preparation method>

An aqueous phase was prepared by dissolving citric acid, sodium citrate, glycerine, 1,3-butylene glycol and trisodium ethylenediaminetetraacetate in purified water.

Separately, an ethanol phase was prepared by dissolving polyoxyethylene (18) oleyl ether, β-carotene and dihydrofarnesol in ethanol.

The ethanol phase was then added to the aqueous phase and the mixture was solubilized, and then filtered to obtain the desired lotion.

A similar preparation was carried out without components (8) and (9) to obtain the lotion in Comparative Example A8 (the same lotion in Comparative Example A5).

### <Test results>

The lotions in Example A8 and Comparative Example A8 were subjected to the sensory evaluation, so that it was found that the group using the lotion in Example A8 showed a significant decrease in the body odor compared to the group using the lotion in Comparative Example A8.

The results are shown in Table A10.

**Table A10**

| Subject No. | Used Composition | Body odor after three days |
|---|---|---|
| 1 | Example A8 | ○ |
| 2 | Example A8 | ○ |
| 3 | Example A8 | ⓞ |
| 4 | Example A8 | ⓞ |
| 5 | Example A8 | ⓞ |
| 6 | Comparative Example A8 | × |
| 7 | Comparative Example A8 | Δ |
| 8 | Comparative Example A8 | Δ |
| 9 | Comparative Example A8 | × |
| 10 | Comparative Example A8 | Δ |

### Example A9 Lotion

| Components | Amount(wt.%) |
|---|---|
| (1) Glycerine | 4.0 |
| (2) 1,3-Butylene glycol | 4.0 |
| (3) Ethanol | 7.0 |
| (4) Polyoxyethylene (18) oleyl ether | 0.5 |
| (5) Citric acid | 0.05 |
| (6) Sodium citrate | 0.1 |
| (7) Trisodium ethylenediaminetetraacetate | 0.02 |
| (8) Dihydrofarnesol | 0.5 |
| (9) Purified water | Residue |

### <Preparation method>

An aqueous phase was prepared by dissolving citric acid, sodium citrate, glycerine, 1,3-butylene glycol, and trisodium ethylenediaminetetraacetate in purified water.

Separately, an ethanol phase was prepared by dissolving dihydrofarnesol and polyoxyethylene(18) oleyl ether in ethanol.

The ethanol phase was then added to the aqueous phase and the mixture was solubilized and filtered to obtain the desired lotion.

A similar preparation was carried out without component(8) to obtain the lotion in Comparative Example A9.

### <Test results>

The lotions in Example A9 and Comparative Example A9 were subjected to the sensory evaluation, so that it was found that the group using the lotion in Example A9 showed a significant decrease in the body odor compared to the group using the lotion in Comparative Example A9.

The results are shown in Table A11.

**Table A11**

| Subject No. | Used Composition | Body odor after three days |
|---|---|---|
| 1 | Example A9 | ○ |
| 2 | Example A9 | ○ |
| 3 | Example A9 | ⓞ |
| 4 | Example A9 | ⓞ |
| 5 | Example A9 | ○ |
| 6 | Comparative Example A9 | × |
| 7 | Comparative Example A9 | Δ |
| 8 | Comparative Example A9 | Δ |
| 9 | Comparative Example A9 | × |
| 10 | Comparative Example A9 | Δ |

### Example A10 Emulsion

| Components | Amount(wt.%) |
|---|---|
| (1) Stearic acid | 1.5 |
| (2) Cetyl alcohol | 0.5 |
| (3) Beeswax | 2.0 |
| (4) Polyoxyethylene(10) monooleate | 1.0 |
| (5) Octyl methoxycinnamate | 2.0 |
| (6) Benzalkonium chloride | 0.2 |
| (7) Sodium hyalurorate | 0.01 |
| (8) Triethanolamine | 0.75 |
| (9) Glycerine | 7.0 |
| (10) Inositol | 0.5 |
| (11) Sodium ethylenediaminehydroxytriacecate | 0.01 |
| (12) Purified water | Residue |

### <Preparation method>

An aqueous phase was prepared by dissolving benzalkonium chloride, sodium hyaluronate, glycerine, inositol and sodium ethylenediaminehydroxytriacetate in purified water, and maintaining at 70°C.

Separately, an oil phase was prepared by mixing the other components together, heating and dissolving, and then maintaining at 70°C.

The oil phase was then added to the aqueous phase and the mixture was subjected to preliminary emulsification, and emulsified using a homogenizing mixer and then quenched while being stirred to obtain the desired emulsion.

A similar preparation was carried out without component(6) to obtain the emulsion in Comparative Example A10.

The emulsions in Example A10 and Comparative Example A10 were fully applied to the upper part of the body, so that the emulsion in Example A10 significantly reduced the body odor of the subjects compared to the emulsion in Comparative Example A10.

### Example A11 Lotion spray

| Components | Amount(wt.%) |
|---|---|
| (Solution formulation) | |
| (1) Zinc p-phenolsulfonate | 2.0 |
| (2) Ethanol | 92.8 |
| (3) 1,3-Butylene glycol | 3.0 |
| (4) Isopropyl myristate | 2.0 |
| (5) Trichlorocarbanilide | 0.2 |

| (Filling formulation) | |
|---|---|
| (6) Solution | 50.0 |
| (7) LPG | 50.0 |

### <Preparation method>

First, the solution components was prepared by dissolving in the ethanol the other components of the solution and filtering, and the solution obtained was then placed in an aerosol container. A valve was then installed in the container and LPG was charged therein to prepare the desired lotion spray.

A similar preparation was carried out without component(5) to obtain the lotion spray in Comparative Example A11.

### <Test results>

The lotion sprays in Example A11 and Comparative Example A11 were subjected to the sensory evaluation, so that it was found that the group using the lotion spray in Example A11 showed a significant decrease in the body odor compared to the group using the lotion spray in Comparative Example A11.

### Example A12 Lotion

| Components | Amount(wt.%) |
|---|---|
| (1) Glycerine | 4.0 |
| (2) 1,3-Butylene glycol | 4.0 |
| (3) Ethanol | 7.0 |
| (4) Polyoxyethylene(18) oleyl ether | 0.5 |
| (5) Citric acid | 0.05 |
| (6) Sodium citrate | 0.1 |
| (7) Trisodium ethylenediaminetetraacetate | 0.02 |
| (8) Vitamin E | 0.5 |
| (9) Dihydrofarnesol | 0.1 |
| (10) Purified water | Residue |

### <Preparation method>

An aqueous phase was prepared by dissolving citric acid, sodium citrate, glycerine, 1,3-butylene glycol and trisodium ethylenediaminetetraacetate in purified water.

Separately, an ethanol phase was prepared by dissolving polyoxyethylene(18) oleyl ether, vitamin E and dihydrofarnesol in ethanol.

The ethanol phase was then added to the aqueous phase and the mixture was solubilized, and filtered to obtain the desired lotion.

A similar preparation was carried without components(8) and (9) to obtain the lotion in Comparative Example A12.

### <Test results>

The lotions in Example A12 and Comparative Example A12 were subjected to the sensory evaluation, so that it was found that the group using the lotion in Example A12 showed a significant decrease in the body odor compared to the group using the lotion in Comparative Example A12.

Table A12 shows the results.

**Table A12**

| Subject No. | Used Composition | Body odor afterr three days |
|---|---|---|
| 1 | Example A12 | ○ |
| 2 | Example A12 | ○ |
| 3 | Example A12 | ⓞ |
| 4 | Example A12 | ⓞ |
| 5 | Example A12 | ⓞ |
| 6 | Comparative Example A12 | × |
| 7 | Comparative Example A12 | Δ |
| 8 | Comparative Example A12 | Δ |
| 9 | Comparative Example A12 | × |
| 10 | Comparative Example A12 | Δ |

### Example A13 Emulsion

| Components | Amount(wt.%) |
|---|---|
| (1) Stearic acid | 1.5 |
| (2) Cetyl alcohol | 0.5 |
| (3) Beeswax | 2.0 |
| (4) Polyoxyethylene(10) monooleate | 1.0 |
| (5) Octyl methoxycinnamate | 2.0 |
| (6) Alanine(antioxidant) | 0.2 |
| (7) Tranexamic acid | 0.5 |
| (8) Sodium hyaluronate | 0.01 |
| (9) Triethanolamine | 0.75 |
| (10) Glycerine | 7.0 |
| (11) Inositol | 0.5 |
| (12) Sodium ethylenediaminehydroxytriacetate | 0.01 |
| (13) Purified water | Residue |

### <Preparation method>

An aqueous phase was prepared by dissolving alanine, tranexamic acid, sodium hyaluronate, glycerine, inositol and sodium ethylenediaminehydroxytriacetate in purified water, and maintaining at 70°C.

Separately, an oil phase was prepared by mixing the other components together, heating and dissolving, and then maintaining at 70°C.

The oil phase was then added to the aqueous phase and the mixture was subjected to preliminary emulsification, and emulsified using a homogenizing mixer and then quenched while being stirred to obtain the desired emulsion.

A similar preparation was carried out without components(6) and (7) to obtain the emulsion in Comparative Example A13 (the same emulsion as obtained in Comparative Example A2).

The emulsions in Example A13 and Comparative Example A13 were fully applied to the upper part of the body, so that the emulsion in Example A13 significantly reduced the body odor of the subjects compared to the emulsion in Comparative Example A13.

### Example A14 Lotion spray

| Components | Amount(wt.%) |
|---|---|
| (Solution formulation) | |
| (1) Zinc p-phenol sulfonate | 2.0 |
| (2) Ethanol | 92.6 |
| (3) 1,3-Butylene glycol | 3.0 |
| (4) Isopropyl myristate | 2.0 |
| (5) Bupleurum extract | 0.2 |
| (6) Tranexamic acid | 0.2 |

| (Filling formulation) | |
|---|---|
| (7) Solution | 50.0 |
| (8) LPG | 50.0 |

### <Preparation method>

First, the solution was prepared by dissolving in ethanol the other components of the solution and filtering. The solution obtained was then placed in an aerosol container. A valve was then installed in the container and LPG was charged therein to prepare the desired lotion spray.

A similar preparation was carried out without components(5) and (6) to obtain the lotion spray in Comparative Example A14.

### <Test results>

The lotion sprays in Example A14 and Comparative Example A14 were subjected to the sensory evaluation, so that it was found that the group using the lotion spray in Example A14 showed a significant decrease in the body odor compared to the group using the lotion spray in Comparative Example A14.

### Example A15 Emulsion

| Components | Amount(wt.%) |
|---|---|
| (1) Stearic acid | 1.5 |
| (2) Cetyl alcohol | 0.5 |
| (3) Beeswax | 2.0 |
| (4) Polyoxyethylene(10) monooleate | 1.0 |
| (5) Octyl methoxycinnamate | 2.0 |
| (6) Benzalkonium chloride | 0.2 |
| (7) Tranexamic acid | 0.5 |
| (8) Sodium hyaluronate | 0.01 |
| (9) Triethanolamine | 0.75 |
| (10) Glycerine | 7.0 |
| (11) Inositol | 0.5 |
| (12) Sodium ethylenediaminehydroxytriacetate | 0.01 |
| (13) Purified water | Residue |

### <Preparation method>

An aqueous phase was prepared by dissolving benzalkoniumu chloride, tranexamic acid, sodium hyaluronate, glycerine, inositol and sodium ethylenediaminehydroxytriacetate in purified water, and maintaining at 70°C.

On the other hand, an oil phase was prepared by mixing the other components together, heating and dissolving, and then maintaining at 70°C.

The oil phase was then added to the aqueous phase and the mixture was subjected to preliminary emulsification, and emulsified using a homogenizing mixer and then quenched while being stirred to obtain the desired emulsion.

A similar preparation was carried out without components (6) and (7) to obtain the emulsion in Comparative Example A15 (the same emulstion as obtained in Comparative Example A10).

The emulsions in Example A15 and Comparative Example A15 were fully applied to the upper part of the body, so that the emulsion in Example A15 significantly reduced the body odor of the subjects, compared to the emulstion in Comparative Example A15.

### Example A16 Lotion spray

| Components | Amount(wt.%) |
|---|---|
| (Solution formulation) | |
| (1) Zinc p-phenol sulfonate | 2.0 |
| (2) Ethanol | 92.6 |
| (3) 1,3-Butylene glycol | 3.0 |
| (4) Isopropyl myristate | 2.0 |
| (5) Trichlorocarbanilide | 0.2 |
| (6) Tranexamic acid | 0.2 |

| (Filling formulation) | |
|---|---|
| (7) Solution | 50.0 |
| (8) LPG | 50.0 |

### <Preparation method>

First, the solution was prepared by dissolving in ethanol the other components of the solution and filtering. The solution obtained was then placed in an aerosol container. A valve was then installed in the container and LPG was charged therein to prepare the desired lotion spray.

A similar preparation was carried out without components (5) and (6) to obtain the lotion spray in Comparative Example A16 (the same spray as obtained in Comparative Example A11).

### <Test results>

The lotion sprays in Example A16 and Comparative Example A16 were subjected to the sensory evaluation, so that it was found that the group using the lotion spray in Example A16 showed a significant decrease in the body odor compared to the group using the lotion spray in Comparative Example A16.

### Example A17 Lotion

| Components | Amount(wt.%) |
|---|---|
| (1) Glycerine | 4.0 |
| (2) 1,3-Butylene glycol | 4.0 |
| (3) Ethanol | 7.0 |
| (4) Polyoxyethylene(18) oleyl ether | 0.5 |
| (5) Citric acid | 0.05 |
| (6) Sodium citrate | 0.1 |
| (7) Trisodium ethylenediaminetetraacetate | 0.02 |
| (8) Culen extract | 0.5 |
| (9) β-Carotene | 0.5 |
| (10) Dihydrofarnesol | 0.1 |
| (11) Purified water | Residue |

### <Preparation method>

An aqueous phase was prepared by dissolving citric acid, sodium citrate, glycerine, 1,3-butylene glycol, Culen extract and trisodium ethylenediaminetetraacetate in purified water.

Separately, an ethanol phase was prepared by dissolving polyoxyethylene (18) oleyl ether, β-carotene and dihydrofarnesol in ethanol.

The ethanol phase was then added to the aqueous phase and the mixture was solubilized, and filtered to obtain the desired lotion.

A similar preparation was carried out without components (8), (9) and (10) to obtain the lotion in Comparative Example A17 (the same lotion as obtained in Comparative Example A1).

### <Test results>

The lotions in Example A17 and Comparative Example A17 were subjected to the sensory evaluation, so that it was found that the group using the lotion in Example A17 showed a significant decrease in the body odor compared to the group using the lotion in Comparative Example A17.

The results shown in Table A13.

**Table A17**

| Subject No. | Used Composition | Body odor after three days |
|---|---|---|
| 1 | Example A13 | ⓞ |
| 2 | Example A13 | ⓞ |
| 3 | Example A13 | ⓞ |
| 4 | Example A13 | ⓞ |
| 5 | Example A13 | ○ |
| 6 | Comparative Example A13 | × |
| 7 | Comparative Example A13 | × |
| 8 | Comparative Example A13 | Δ |
| 9 | Comparative Example A13 | × |
| 10 | Comparative Example A13 | Δ |

### Example A18 Emulsion

| Components | Amount(wt.%) |
|---|---|
| (1) Stearic acid | 1.5 |
| (2) Cetyl alcohol | 0.5 |
| (3) Beeswax | 2.0 |
| (4) Polyoxyethylene(10) monooleate | 1.0 |
| (5) Octyl methoxycinnamate | 2.0 |
| (6) Scutellaria root extract | 0.2 |
| (7) Tranexamic acid | 0.5 |
| (8) Dihydrofarnesol | 0.2 |
| (9) Sodium hyaluronate | 0.01 |
| (10) Triethanolamine | 0.75 |
| (11) Glycerine | 7.0 |
| (12) Inositol | 0.5 |
| (13) Sodium ethylenediaminehydroxytriacetate | 0.01 |
| (14) Purified water | Residue |

### <Preparation method>

An aqueous phase was prepared by dissolving Scutellaria root extract, tranexamic acid, sodium hyaluronate, glycerine, inositol and sodium ethylenediaminehydroxytriacetate in purified water, and maintaining at 70°C.

Separately, an oil phase was prepared by mixing the other components together, heating and dissolving, and then maintaining at 70°C.

The oil phase was then added to the aqueous phase and the mixture was subjected to preliminary emulsification, and emulsified using a homogenizing mixer and then quenched while being stirred to obtain the desired emulsion.

A similar preparation was carried out without components (6), (7) and (8) to obtain the emulsion in Comparative Example A18 (the same emulstion as obtained in Comparative Example A2).

The emulsions in Example A18 and Comparative Example A18 were fully applied to the upper part of the body, so that the emulsion in Example A18 significantly reduced the body odor of the subjects compared to the emulsion in Comparative Example A18.

### Example A19 Lotion spray

| Components | Amount(wt.%) |
|---|---|
| (Solution formulation) | |
| (1) Zinc p-phenolsulfonate | 2.0 |
| (2) Ethanol | 92.4 |
| (3) 1,3-Butylene glycol | 3.0 |
| (4) Isopropyl myristate | 2.0 |
| (5) Bupleurum extract | 0.2 |
| (6) Tranexamic acid | 0.2 |
| (7) Dihydrofarnesol | 0.2 |

| (Filling formulation) | |
|---|---|
| (8) Solution | 50.0 |
| (9) LPG | 50.0 |

### <Preparation method>

First, the solution was prepared by dissolving in ethanol the other components of the solution, and filtering. The solution obtained was then placed in an aerosol container. A valve was then installed in the container and LPG was charged therein to prepare the desired lotion spray.

A similar preparation was carried out without components(5), (6) and (7) to obtain the lotion spray in Comparative Example A19 (the same spray as obtained in Comparative Example A3).

### <Test results>

The lotion sprays in Example A19 and Comparative Example A19 were subjected to the sensory evaluation, so that it was found that the group using the lotion spray in Example A19 showed a significant decrease in the body odor compared to the group using the lotion spray in Comparative Example A19.

The above results shows that the composition ontaining antioxidants, lipoxygenase inhibitors or antibacterial agents solely or in combination can efficiently remove the body odor such as the aged body odor as an external agent for the skin. The above results additionally shows that the composition of the present invention containing the above components in combination has more excellent effects of preventing the body odor.

### B. Examples of Composition (B)

### Test Example B1

### Aged body odor preventing test

Aged body odor preventing test was conducted as described below to evaluate the masking and harmonizing effects on the aged body odor of test components.

Ten µL of a test component was added to 100 µL of the composition causing the aged body odor shown in Table B1 below, to give a mixture, and a filter paper(2 cm x 2 cm) was impregnated with the mixture. The filter paper was placed in a paper cup(500 mL) having coated inner surface, and the mouth of the paper cup was sealed with aluminum foil. Thirty minutes later, a hole as large as a pencil was made in the aluminum foil to sensoryly evaluate on the masking and harmonizing effects on the aged body odor.

The evaluation values for the masking and harmonizing effects are shown as the average of numerical values provided by eight panelists based on the results of sensory evaluations using the following evaluation criteria.

### (1) Evaluation criteria for the masking effect

| | |
|---|---|
| Masked very well | 3 |
| Masked well | 2 |
| Slightly masked | 1 |
| Not masked | 0 |

### (2) Evaluation criteria for the harmonizing effect

| | |
|---|---|
| Harmonizing | 1 |
| Unclear | 0 |
| Not harmonizing | -1 |

**Table B1**

| Composition causing the aged body odor | Wt.% |
|---|---|
| Dibutylhydroxytoluene | 1.0 |
| Heptanoic acid | 10.0 |
| Octanoic acid | 10.0 |
| Triethyl citrate containing 1% nonenal | 15.0 |
| Triethyl citrate containing 1% octenal | 2.5 |
| Triethyl citrate | 61.5 |

### Evaluation of Masking Effect

In this evaluation, a test component receiving a score of 2.6 or higher for the masking effect was considered to be a "component having an excellent masking effect on the aged body odor", and a test component receiving a score of 2.7 or higher was considered to be a "component having an extremely excellent masking effect on the aged body odor".

### Masking effect test (1)

| Test components | Evaluation value |
|---|---|
| Armoise oil | 3.0 |
| Basil oil | 2.6 |
| Galbanum oil | 2.8 |
| Geranium oil | 2.7 |
| Spearmint oil | 2.9 |
| Ylang ylang oil | 2.9 |
| Cardamon oil | 2.7 |
| Elemi oil | 2.8 |
| Lavender oil | 2.7 |
| Peppermint oil | 3.0 |
| Nutmeg oil | 2.6 |
| Chamomile oil | 2.8 |
| Eucalyptus oil | 2.8 |
| Rosemary oil | 2.9 |
| Allylamyl glycolate | 2.8 |
| 1,1-Dimethoxy-2-phenylethane | 2.6 |
| 2,4-Dimethyl-4-phenyltetrahydrofuran | 2.6 |
| Dimethyltetrahydrobenzaldehyde | 2.9 |
| 2,6-Dimethyl-7-octen-2-ol | 2.7 |
| 1,2-Diethoxy-3,7-dimethyl-2,6-octadiene | 2.7 |
| Phenylacetaldehyde | 3.0 |
| Rose oxide | 2.9 |
| Ethyl 2-methylpentanoate | 2.8 |

Each of these test components had an evaluation value of at least 2.6, and thus it can be recognized that these components are all a component having an excellent or extremely excellent masking effect on the aged body odor.

### Masking effect test (2)

This test examined whether well-known sweat odor masking perfumes had a masking effect on the aged body odor.

| Test components | Evaluation value |
|---|---|
| Ionone | 1.3 |
| Isobutyl quinoline | 1.1 |
| Hexyl salicylate | 0.4 |
| 1,3,4,6,7,8-Hexahydro-4,6,6,7,8,8-hexamethylcyclopenta-γ-2-benzopyrane | 0.9 |
| Acetyl cedrine | 0.6 |
| Menthol | 0.3 |

The results shows that the components having a masking effect on sweat odor are totally different from the components having a masking effect on the aged body odor.

### Evaluation of Harmonizing Effect

In this test, a test component receiving a score of 0.6 or higher for the harmonizing effect was considered to be a "component having an excellent harmonizing effect on the aged body odor", and a test component receiving a score of 0.8 or higher was considered to be a "component having an extremely excellent harmonizing effect on aged body odor".

### Harmonizing effect test (1)

| Test components | Evaluation value |
|---|---|
| Lavandin oil | 0.8 |
| Lemon oil | 0.8 |
| Lime oil | 0.9 |
| Orange oil | 0.9 |
| Petitgrain oil | 0.8 |
| Bergamot oil | 1.0 |
| Clary sage oil] | 0.6 |
| Coriander oil | 0.7 |
| Cypress oil | 0.9 |
| Jasmine absolute | 0.9 |
| Rose oil | 0.8 |
| Rose absolute | 0.8 |
| 1-(2,6,6-Trimethyl-1,3-cyclohexadien-1-yl)-2-buten-1-one | 0.8 |
| Ethyl vanillin | 0.8 |
| Mixture of 2,6-dimethyl-2-octenol and 3,7-dimethyl-2-Octenol | 0.8 |
| tert-Butyl cyclohexylacetate | 0.7 |
| Anisyl acetate | 0.6 |
| Allyl cyclohexyloxyacetate | 0.9 |
| Ethyl linalool | 0.7 |
| Eugenol | 0.9 |
| Coumarin | 0.6 |
| Ethyl acetacetate | 0.7 |
| 4-Phenyl-2,4,6-trimethyl-1,3-dioxane | 0.9 |
| Ethyl acetacetate ethylene glycol ketal | 0.7 |
| 4-Methylene-3,5,6,6-tetramethyl-2-heptanon | 0.8 |
| Ethyl tetrahydrosafranate | 1.0 |
| Geranyl nitrile | 0.7 |
| Cis-3-hexen-1-ol | 0.9 |
| Cis-3-hexenyl acetate | 0.9 |
| Cis-3-hexenyl methyl carbonate | 0.7 |
| 2,6-Dimethyl-5-hepten-1-al | 0.6 |
| 4-(Tricyclo[5.2.1.0 ^{2,6} ]decylidene)-8-butanal | 0.8 |
| 5-(2,2,3-Trimethyl-3-cyclopentenyl)-3-methylpentan-2-ol | 0.6 |
| p-tert-Butyl-α-methylhydrocinnamic aldehyde | 0.8 |
| Ethyl [5.2.1.0 ^{2,6} ] tricyclodecane carboxylate | 1.0 |
| Geraniol | 0.9 |
| Citronellol | 0.9 |
| Citral | 0.7 |

Each of these test components had an evaluation value of at least 0.6 and thus it can be recognized that these components are all a component having an excellent or extremely excellent harmonizing effect on the aged body odor.

### Harmonizing effect test (2)

This test examined whether well-known sweat odor harmonizing perfumes had a harmonizing effect on the aged body odor.

| Test components | Evaluation value |
|---|---|
| Sandalwood oil | 0.1 |
| Clove but oil | 0 |
| Vetiver oil | -0.5 |
| Oakmoss absolute | -0.5 |
| Cedryl acetate | -0.3 |
| Trichloromethyl phenyl carbinyl acetate | -0.5 |
| Undecylenic aldehyde | 0.1 |

The results shows that the components having a harmonizing effect on sweat odor are totally different from the components having a harmonizing effect on the aged body odor.

Examples of formulations of the composition (B) are described below. These compositions were prepared using a known method.

### Example B1

### Female perfume[citrus-floral type]

| Mixed Components | Weight (g) |
|---|---|
| Allylamyl glycolate | 3 |
| Bergamot oil | 20 |
| Citronellol | 450 |
| Coumarin | 3 |
| Damacenone | 1 |
| Ethyl acetoacetate | 25 |
| Ethyl linalool | 100 |
| Ethyl vanillin | 2 |
| Eugenol | 20 |
| Galbanum oil | 1 |
| Cis-3-hexen-1-ol | 2 |
| Cis-3-hexenyl acetate | 1 |
| Triethyl citrate solution containing 50% phenylacetaldehyde | 1 |
| Lemon oil | 120 |
| p-tert-butyl-α-methyl hydrocinnamic aldehyde | 50 |
| Orange oil | 80 |
| Petigrain oil | 50 |
| Sandalore (5-(2,2,3-trimethyl-3-cyclopentenyl)-3-methylpentan-2-ol) | 10 |
| Tesalon (ethyl tetrahydrosafranate) | 5 |
| Trypral (dimethyltetrahydrobenzaldehyde) | 1 |
| Ylang ylang oil | 25 |
| Dihydrofarnesol | 30 |
| | Total 1000 |

The evaluation values for the female perfume were 3.0 in the evaluation test of the masking effect and 1.0 in the evaluation test of the harmonizing effect. Therefore, it was clarified that the perfume had extremely excellent masking and harmonizing effects on the aged body odor.

A female eau de Toilette which contained an appropriate amount of the female perfume, which eau de Toilette was also the composition of the present invention, had an excellent effect of preventing the aged body odor by the masking and harmonizing effects.

### Example B2

### Male or unisex perfume[citrus-green-floral type]

| Mixed components | Weight (g) |
|---|---|
| Allylamyl glycolate | 10 |
| Bergamot oil | 200 |
| Clary sage oil | 10 |
| Dihydromylcenol (2,6-dimethyl-7-octen-2-ol) | 100 |
| p-Ethyl-α,α-dimethyl hydrocinnamic aldehyde | 5 |
| Geraniol | 20 |
| Geranium oil | 10 |
| Cis-3-hexen-1-ol | 10 |
| Lavender oil | 20 |
| p-tert-butyl-α-methyl hydrocinnamic aldehyde | 100 |
| Linacsol (Mixture of 2,6-dimethyl-2-octenol and 3,7-dimethyl-3-octenol) | 100 |
| Orange oil | 350 |
| Sandalore (5-(2,2,3-trimethyl-3-cyclopentenyl)-3-methylpentan-2-ol) | 10 |
| Spearmint oil | 5 |
| Dihydrofarnesol | 50 |
| | Total 1000 |

The evaluation values for the male or unisex perfume were 3.0 in the evaluation test of the masking effect and 1.0 in the evaluation test of harmonizing effect. Therefore, it was clarified that the perfume had extremely excellent masking and harmonizing effects on the aged body odor.

A soap which contained an appropriate amount of the perfume, which soap is also the composition of the present invention, had an excellent effect of preventing the aged body odor by the masking and harmonizing effects.

### Example B3

### Perfume for preparation of aromatics (lavender type)

| Mixed Components | Weight (g) |
|---|---|
| Eucalyptus oil | 20 |
| Bergamot oil | 30 |
| Geranium oil | 10 |
| Lavender oil | 150 |
| Lavandin oil | 250 |
| Nutmeg oil | 5 |
| Allylamyl glycolate | 2 |
| Cis-3-hexenol | 1 |
| Cis-3-hexenyl acetate | 3 |
| Dihydromylcenol | 30 |
| Geraniol | 5 |
| Tapineol | 10 |
| Linalool | 130 |
| Linalyl acetate | 150 |
| Dihydrofarnesol | 30 |
| Dipropylene glycol | 174 |
| | Total 1000 |

The evaluation values for the perfume for preparing aromatics were 3.0 in the evaluation test of the masking effect and 1.0 in the evaluation test of the harmonizing effect. Therefore, it was clarified that the perfume had extremely excellent masking and harmonizing effects on the aged body odor.

An aromatic which contains an appropriate amount of the perfume, which aromatic is also the composition of the present invention, had an excellent effect of preventing the aged body odor by the masking and harmonizing effects.

Thus, according to the present invention, there is provided a composition of preventing the body odor, representatively the aged body odor, which has a function of preventing the generation of the body odor and/or a function of sensoryly preventing the body odor.

## Claims

1. A method of preventing aged body odor containing octenal and/or nonenal, comprising the step of applying to the skin and/or hair and/or clothes of an aged person whose body secretes octenal and/or nonenal, and/or introducing into air or space where the aged body odor may be present an effective amount of a composition comprising
(A) at least one selected from the group consisting of an antioxidant, a lipoxygenase inhibitor and an antibacterial agent, and/or
(B) a compound for masking the aged body odor and/or a component for harmonizing the aged body odor.

2. A method according to claim 1 wherein said composition comprises (B) a component for masking the aged body odor and/or a component for harmonizing the aged body odor.

3. The method according to claim 2, wherein the component for masking the aged body odor is at least one selected from the group consisting of armoise oil, basil oil, galbanum oil, geranium oil, spearmint oil, ylang ylang oil, cardamom oil, elemi oil, lavender oil, peppermint oil, nutmeg oil, chamomile oil, eucalyptus oil, rosemary oil, allylamyl glycolate, 1,1-dimethoxy-2-phenylethane, 2,4-dimethyl-4-phenyltetrahydrofuran, dimethyltetrahydrobenzaldehyde, 2,6-dimethyl-7-octen-2-ol, 1,2-diethoxy-3,7-dimethyl-2,6-octadiene, phenylacetaldehyde, rose oxide and ethyl 2-methylpentanoate.

4. The method according to claim 2, wherein the component for harmonizing the aged body odor is at least one selected from the group consisting of lavandin oil, lemon oil, lime oil, orange oil, petitgrain oil, bergamot oil, clary sage oil, coriander oil, cypress oil, jasmine absolute, rose oil, rose absolute, 1-(2,6,6-trimethyl-1,3-cyclohexadien-1-yl)-2-buten-1-one, ethyl vanillin, a mixture of 2,6-dimethyl-2-octenol and 3,7-dimethyl-2-octenol, tert-butyl cyclohexylacetate, anisyl acetate, allyl cyclohexyloxyacetate, ethyl linalool, eugenol, coumarin, ethyl acetacetate, 4-phenyl-2,4,6-trimethyl-1,3-dioxane, ethyl acetacetate ethylene glycol ketal, 4-methylene-3,5,6,6-tetramethyl-2-heptanone, ethyl tetrahydrosafranate, geranyl nitrile, cis-3-hexen-1-ol, cis-3-hexenyl acetate, cis-3-hexenyl methyl carbonate, 2,6-dimethyl-5-hepten-1-al, 4-(tricyclo[5.2.1.0^{2,6}] decylidene)-8-butanal, 5-(2,2,3-trimethyl-3-cyclopentenyl)-3-methylpentan-2-ol, p-tert-butyl-α-methylhydrocinnamic aldehyde, ethyl[5.2.1.0^{2,6}]tricyclodecane carboxylate, geraniol, citronellol and citral.

## Patentansprüche

1. Verfahren zur Verhütung des Körpergeruchs älterer Leute, der Octenal und/oder Nonenal enthält, das die Stufe der Applikation einer wirksamen Menge einer Zusammensetzung, die
(A) mindestens einen Bestandteil, der aus der Gruppe von einem Antioxidationsmittel, einem Lipoxygenaseinhibitor und einem antibakteriellen Mittel ausgewählt ist, und/oder
(B) eine Verbindung zur Maskierung des Körpergeruchs älterer Leute und/oder eine Komponente zur Harmonisierung des Körpergeruchs älterer Leute
umfasst, auf die Haut und/oder das Haar und/oder die Kleider einer älteren Person, deren Körper Octenal und/oder Nonenal absondert, und/oder der Stufe des Abgebens derselben an die Luft oder den Raum, wo der Körpergeruch älterer Leute vorhanden sein kann, umfasst.

2. Verfahren nach Anspruch 1, wobei die Zusammensetzung (B) eine Komponente zur Maskierung des Körpergeruchs älterer Leute und/oder eine Komponente zur Harmonisierung des Körpergeruchs älterer Leute umfasst.

3. Verfahren nach Anspruch 2, wobei die Komponente zur Maskierung des Körpergeruchs älterer Leute mindestens eine ist, die ausgewählt ist aus der Gruppe von Beifußöl, Basilikumöl, Galbanumöl, Geraniumöl, Spearmintöl, Ylang-Ylang-Öl, Kardamomöl, Elemiöl, Lavendelöl, Pfefferminzöl, Muskatnussöl, Kamillenöl, Eukalyptusöl, Rosmarinöl, Allylamylglykolat, 1,1-Dimethoxy-2-phenylethan, 2,4-Dimethyl-4-phenyltetrahydrofuran, Dimethyltetrahydrobenzaldehyd, 2,6-Dimethyl-7-octen-2-ol, 1,2-Diethoxy-3,7-dimethyl-2,6-octadien, Phenylacetaldehyd, Rosenoxid und Ethyl-2-methylpentanoat.

4. Verfahren nach Anspruch 2, wobei die Komponente zur Harmonisierung des Körpergeruchs älterer Leute mindestens eine Komponente ist, die ausgewählt ist aus der Gruppe von Lavandinöl, Citronenöl, Limettöl, Orangenöl, Bitterorangenöl, Bergamottöl, Muskatellersalbeiöl, Corianderöl, Cypressenöl, Jasminabsolue, Rosenöl, Rosenabsolue, 1-(2,6,6-Trimethyl-1,3-cyclohexadien-1-yl)-2-buten-1-on, Ethylvanillin, einem Gemisch von 2,6-Dimethyl-2-octenol und 3,7-Dimethyl-2-octenol, tert.-Butylcyclohexylacetat, Anisylacetat, Allylcyclohexyloxyacetat, Ethyllinalool, Eugenol, Cumarin, Ethylacetacetat, 4-Phenyl-2,4,6-trimethyl-1,3-dioxan, Ethylacetacetatethylenglykolketal, 4-Methylen-3,5,6,6-tetramethyl-2-heptanon, Ethyltetrahydrosafranat, Geranylnitril, cis-3-Hexen-1-ol, cis-3-Hexenylacetat, cis-3-Hexenylmethylcarbonat, 2,6-Dimethyl-5-hepten-1-al, 4-(Tricyclo[5.2.1.0^{2,6}]decyliden)-8-butanal, 5-(2,2,3-Trimethyl-3-cyclopentenyl)-3-methylpentan-2-ol, p-tert.-Butyl-α-methylhydrozimtaldehyd, Ethyl-[5.2.1.0^{2,6}]tricyclodecancarboxylat, Geraniol, Citronellol und Citral.

## Revendications

1. Méthode de prévention d'une odeur corporelle, liée à l'âge, contenant de l'octénal et/ou du nonénal, ladite méthode comprenant l'étape qui consiste à appliquer, sur la peau et/ou les cheveux et/ou les vêtements d'une personne âgée dont le corps sécrète de l'octénal et/ou du nonénal, et/ou à introduire, dans l'air ou l'espace où cette odeur corporelle liée à l'âge est susceptible d'être présente, une quantité efficace d'une composition comprenant :
(A) au moins un composant choisi dans le groupe constitué par un antioxydant, un inhibiteur de la lipoxygénase et un agent antibactérien, et/ou
(B) un composé destiné à dissimuler l'odeur corporelle liée à l'âge et/ou un composant destiné à harmoniser l'odeur corporelle liée à l'âge.

2. Méthode selon la revendication 1, dans laquelle ladite composition comprend (B) un composant destiné à dissimuler l'odeur corporelle liée à l'âge et/ou un composant destiné à harmoniser l'odeur corporelle liée à l'âge.

3. Méthode selon la revendication 2, dans laquelle le composant destiné à dissimuler l'odeur corporelle liée à l'âge est au moins un composant choisi dans le groupe constitué par l'essence d'armoise, l'essence de basilic, l'essence de galbanum, l'essence de géranium, l'essence de menthe verte, l'essence d'ylang ylang, l'essence de cardamome, l'essence d'élémi, l'essence de lavande, l'essence de menthe poivrée, l'essence de noix de muscade, l'essence de camomille, l'essence d'eucalyptus, l'essence de romarin, le glycolate d'allylamyle, le 1,1-diméthoxy-2-phényléthane, le 2,4-diméthyl-4-phényltétrahydrofuranne, le diméthyltétrahydro-benzaldéhyde, le 2,6-diméthyl-7-octén-2-ol, le 1,2-diéthoxy-3,7-diméthyl-2,6-octadiène, le phénylacétaldéhyde, l'oxyde de rose et le 2-méthylpentanoate d'éthyle.

4. Méthode selon la revendication 2, dans laquelle le composant destiné à harmoniser l'odeur corporelle liée à l'âge est au moins un composant choisi dans le groupe constitué par l'essence de lavandin, l'essence de citron, l'essence de citron vert, l'essence d'orange, l'essence de petit-grain, l'essence de bergamote, l'essence de sauge sclarée, l'essence de coriandre, l'essence de cyprès, l'absolu de jasmin, l'essence de rose, l'absolu de rose, la 1-(2,6,6-triméthyl-1,3-cyclohexadièn-1-yl)-2-butén-1-one, l'éthylvanilline, un mélange de 2,6-diméthyl-2-octénol et de 3,7-diméthyl-2-octénol, le cyclohexylacétate de tert-butyle, l'acétate d'anisyle, le cyclohexyloxyacétate d'allyle, le linalol d'éthyle, l'eugénol, la coumarine, l'acétacétate d'éthyle, le 4-phényl-2,4,6-triméthyl-1,3-dioxanne, le cétal d'éthylène glycol et d'acétacétate d'éthyle, la 4-méthylène-3,5,6,6-tétraméthyl-2-heptanone, le tétra-hydrosafranate d'éthyle, le nitrile de géranyle, le cis-3-hexén-1-ol, l'acétate de cis-3-hexényle, le carbonate de cis-3-hexénylméthyle, le 2,6-diméthyl-5-heptén-1-al, le 4-(tricyclo[5.2.1.0^{2,6}]décylidène)-8-butanal, le 5-(2,2,3-triméthyl-3-cyclopentényl)-3-méthylpentan-2-ol, l'aldéhyde p-tert-butyl-α-méthylhydrocinnamique, le carboxylate d'éthyl[5.2.1.0^{2,6}]tricyclodécane, le géraniol, le citronellol et le citral.
